(19) Europäisches Patentamt
European Patent Office
Office européen des brevets

(11) EP 1 212 421 B1

(12) EUROPEAN PATENT SPECIFICATION

(45) Date of publication and mention of the grant of the patent: **09.11.2005 Bulletin 2005/45**

(21) Application number: **00962353.9**

(22) Date of filing: **21.08.2000**

(51) Int Cl.[7]: **C12N 15/12**, C12N 5/10, C07K 14/47, C07K 16/18, C12Q 1/68, G01N 33/50, A61K 31/70, A61K 38/17, A61P 25/00

(86) International application number: **PCT/EP2000/008182**

(87) International publication number: **WO 2001/014549 (01.03.2001 Gazette 2001/09)**

(54) **GAS1 POLYPEPTIDES**

GAS1-POLYPEPTIDE

POLYPEPTIDES GAS1

(84) Designated Contracting States:
**AT BE CH CY DE DK ES FI FR GB GR IE IT LI LU MC NL PT SE**
Designated Extension States:
**AL LT LV MK RO SI**

(30) Priority: **24.08.1999 EP 99306702**

(43) Date of publication of application: **12.06.2002 Bulletin 2002/24**

(73) Proprietor: **JANSSEN PHARMACEUTICA N.V.**
**2340 Beerse (BE)**

(72) Inventors:
- **LUYTEN, Walter Herman Maria Louis**
  **2340 Beerse (BE)**
- **NARANJO, Jose Ramon**
  **Cantoblanco, 28049 Madrid (ES)**
- **MELLSTRÖM, Britt**
  **Cantoblanco, 28049 Madrid (ES)**

(56) References cited:
- **EVDOKIOU A. & COWLED P.A.: "Growth-regulatory activity of the growth arrest-specific gene, gas1, in NIH3T3 fibroblasts" EXP. CELL RES., vol. 240, 1 May 1998 (1998-05-01), pages 359-367, XP000892323**
- **LEE T.C. ET AL.: "Myc represses transcription of the growth arrest gene gas1." PROC. NATL. ACAD. SCI. USA, vol. 94, November 1997 (1997-11), pages 12886-12891, XP002133329**
- **DEL SAL G. ET AL.: "Gas1-induced growth suppression requires a transactivation-independent p53 function" MOL CELL. BIOL., vol. 15, no. 12, December 1995 (1995-12), pages 7152-7160, XP002156391**
- **RUARO E. ET AL.: "A proline-rich motif in p53 is required for transactivation-independent growh arrest as induced by Gas1" PROC. NATL. ACAD. SCI. USA, vol. 94, April 1997 (1997-04), pages 4675-4680, XP002133332**
- **DEL SAL G. ET AL.: "The growth arrest-specific gene, gas1, is involved in growth suppression" CELL, vol. 70, 21 August 1992 (1992-08-21), pages 595-607, XP002133330 cited in the application**
- **DEL SAL G. ET AL.: "Structure, function and chromosomal mapping of the growth-suppressing human homologue of the murine gas1 gene" PROC. NATL. ACAD. SCI. USA, vol. 91, March 1994 (1994-03), pages 1848-1852, XP002133331 cited in the application**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 212 421 B1

Printed by Jouve, 75001 PARIS (FR)

## Description

The present invention is concerned with methods of identifying compounds capable of preventing or accelerating Gas1 mediated neuronal cell death.

The Gas1 gene encodes a membrane protein which has been associated with the G0 phase of proliferative arrest and cell cycle exit in rat fibroblasts deprived of serum. Based upon its antiproliferative effects and its functional dependency to p53, Gas1 has also been associated with antitumour like activity. (Schneider et al., 1988 Genes specifically expressed at growth arrest in mammalian cells. Cell 54:787-793; Del Sal *et al*, 1992, the growth arrest specific gene, Gas1, is involved in growth suppression, Cell 70:593-607;' Del Sal e*t al*, 1994. Structure, function and chromosome mapping of the growth suppressing human homologue of the murine Gas1 gene, Proc. Natl. Acad. Sci. USA 91; 1848-1852). The structural conformation of the Gas1 protein deduced from the amino acid sequence thereof, indicates the presence of two transmembrane segments. Except for a hypothetical RGD domain (Arginine-Glycine-Aapartic acid) which is known to interact with integrines , Gas1 does not show any described domain that relates to its cell cycle arrest function. Recently, mRNA Gas1 induction during involution of the prostate, mammary gland and the ovarian luteal body, as a result of castration, lactation and birth delivery arrest, respectively, has been observed (Jaggi et al., 1996, Regulation of a Physiological Apoptosis: Mouse mammary Involution. J. Davy Sci. 79: 1074-1084).

From expression studies, the present inventors have surprisingly found that Gas1 overexpression induces cell death in various cell types, such as, neurons and neuroblastoma cell lines and that Gas1 is responsible for the induction of apoptotic activity in a neuronal cell.

Based upon the surprising relationship of Gas1 expression and apoptosis the present inventors have developed methods to study the effects of expressing proteins in a cell which are normally lethal to the cell by inhibiting expression or activity of either the Gas1 protein or a protein in the signal transduction pathway of which Gas1 is a component. These methods can be further applied to identifying compounds which inhibit or enhance the expression of those otherwise lethal proteins. An assay has also been developed to identify compounds which are capable of preventing or accelerating Gas1 mediated cell death.

Therefore in accordance with a first aspect of the invention there is provided, an in vitro method of inhibiting the lethal effect of expressing an otherwise lethal protein in a neuronal cell, said method comprising (a) providing a neuronal cell or neuronal tissue having (i) a nucleotide sequence encoding a Gas1 protein, or a Gas1 derivative, which is capable of inducing apoptosis in said cell and (ii) a further nucleotide sequence encoding said otherwise lethal protein; (b) inhibiting function and/or expression of said Gas1 protein or Gas1 derivative; and (c) expressing said sequence encoding said otherwise lethal protein.

Thus, advantageously, it is now possible, by inhibiting the function or expression of the biological mediator of cell death to study proteins which are normally lethal when expressed in a neuronal cell. These methods can also be used to identify compounds which can function as enhancers/inhibitors of expression or activity of the otherwise lethal proteins and which has not been possible hitherto.

Therefore, according to a second aspect of the present invention there is provided an in vitro method of identifying compounds which inhibit or enhance expression or activity of proteins which are otherwise lethal to a neuronal cell, said method comprising (a) providing a neuronal cell or neuronal tissue comprising a nucleotide sequence encoding a Gas1 protein or Gas1 derivative, which is capable of inducing apoptosis in said cell, and ii) a further sequence encoding said otherwise lethal protein; (b) inhibiting function and/or expression of said Gas1 protein or Gas1 derivative or a protein in the apoptotic pathway of which Gas1 is a component; (c) expressing said sequence encoding said otherwise lethal protein; (d) contacting said cell with a compound to be tested; and (e) monitoring the effect of said compound on said otherwise lethal protein compared to an identical cell which has not been contacted with said compound.

Preferably, the inhibition of activity or expression of the Gas1 protein occurs by providing a nucleic acid molecule, such as an antisense molecule, which is capable of hybridising to mRNA in the cell corresponding to or complementary to Gas1 DNA under stringent conditions, to prevent expression thereof. The nucleic acid molecule in addition to possessing antisense activity, may in some embodiments possess ribozyme or DNAzyme activity.

The methods of the present invention, therefore, involve inhibiting the function or expression of a Gas1 protein *in vivo* using, for example, antisense technology. Antisense technology can be used to control gene expression through triple-helix formation or antisense DNA or RNA, both of which methods are based on binding of a polynucleotide to DNA or RNA. For example, the part of the DNA sequence coding for the mature protein of the present invention is used to design an antisense RNA oligonucleotide of from 10 to 50 base pairs in length. A DNA oligonucleotide is designed to be complementary to a region of the gene involved in transcription (triple-helix - see Lee et al. Nucl. Acids. Res., 6:3073 (1979); Cooney *et al*., Science, 241:456 (1988); and Dervan *et al*., Science, 251: 1360 (1991), thereby preventing transcription and the production of Gas1. The antisense RNA oligonucleotide hybridises to the mRNA *in vivo* and blocks translation of an mRNA molecule into the mature protein. Thus, an non-human animal having expression of the Gas1 protein inhibited may be utilised as a model for expression of otherwise lethal proteins in accordance with the methods of the invention and for identifying potential therapeutic agents capable of inhibiting or enhancing expression or activity of the otherwise lethal proteins.

Preferably, the antisense molecule comprises a specific Gas1 antisense oligonucleotide (SEQ ID 5) which the present inventors have confirmed as being able to block the transduction process of the Gas1 protein and, as described in more detail below, involves a total blocking of the NMDA induced neuron death phenomenon in primary neuron cultures (survival practically of 100%, 25mM Agl column in Fig. 2a) or by staurosporin (200nM) (Fig. 2b). Alternatively, a sequence complementary to the nucleotide sequence encoding Gas1 as identified in SEQ ID No. 3 may be used to prevent expression of the Gas1 protein.

In an even further embodiment it is also possible to inhibit Gas1 expression or activity by, for example, inhibiting expression or activity of a protein which is involved in the pathway of which Gas1 is a component. Thus, using the methods described it is possible to prevent the apoptotic properties of Gas1 proteins being realised either by inhibiting, for example, the signal transduction pathway upstream or downstream of Gas1 DNA to prevent the apoptotic response. It has also been identified that induction of Gas1 leads to a simultaneous increase in calcium concentration within the cell. In the method according to the invention a suitable stimulus can be applied which is lethal to a cell, to induce transcription of the Gas1 protein.

In one embodiment of the methods described herein, the otherwise lethal protein may be provided on a suitable expression vector. Suitable vectors are well known to those of skill in the art. Similarly, the sequence of the further nucleotide sequence encoding said otherwise lethal protein may be provided in an expression vector under the control of suitable regulatory control elements. Such lethal proteins include, for example, glutamate receptors such as any of the type 1 to 8 metabotropic receptors, or NMDA, AMPA or kainate receptors. Alternatively, the lethal protein may comprise a highly expressed recombinant protein which can frequently be toxic to a cell within which it is expressed. Thus, the present invention also, advantageously, provides a system to enable harvesting of highly expressed recombinant proteins.

The nucleic acid molecule utilised in accordance with the methods of the invention to inhibit expression of the Gas1 protein may be provided as an oligonucleotide, which is transformed or transfected into the cell, using techniques well known to those of skill in the art. Alternatively, the sequence may be encoded by a suitable sequence provided in a vector, which is transformed or transfected into the cell, tissue or organism.

Furthermore, the present invention relates to a method of producing an antagonist or agonist of Gas1 according to the invention comprising the steps of any one of the above described screening methods; and additionally (i) synthesizing the compound obtained or identified in said method or a physiologically acceptable analog or derivative thereof in an amount sufficient to provide said antagonist or agonist in a therapeutically effective amount to a patient; and/or (ii) combining the compound obtained or identified in said method or an analog or derivative thereof with a pharmaceutically acceptable carrier.

A further aspect of the invention comprises a method of monitoring the severity of a disease condition mediated by cellular apoptosis in a neuronal cell, or neuronal tissue comprising measuring the level of transcription, expression or activity of a Gas1 protein or Gas1 derivative in said cell or tissue.

The present inventors have also now, advantageously, identified the sequence of the rat Gas1 polypeptide and which has never before been fully characterised. Accordingly, a further aspect of the present invention comprises a nucleic acid molecule encoding a rat Gas1 protein or a Gas1 derivative, comprising an amino acid sequence according to sequence ID No. 2. Preferably, the nucleic acid molecule is a DNA molecule and even more preferably a cDNA molecule, which in a preferred embodiment comprises the sequence of nucleotides according sequence ID No. 1. Alternatively, the invention provides in a further aspect, a nucleic acid molecule encoding a protein capable of inducing apoptosis in a cell, comprising an amino acid according to Sequence ID No. 4 or a nucleic acid molecule which is complementary thereto.

The invention also provides an antisense molecule capable of hybridising to a nucleic acid molecule according to the invention under conditions of high stringency.

Stringency of hybridisation as defined herein refers to conditions under which polynucleic acids are stable. The stability of hybrids is reflected in the melting temperature (Tm) of the hybrids. Tm can be approximated by the formula:

$$81.5°C-16.6\ (\log10[Na^+]+0.41\ (\%G\&C)-600/1$$

wherein 1 is the length of the hybrids in nucleotides. Tm decreases approximately by 1-1.5°C with every 1% decrease in sequence homology.

Preferably, the antisense molecule comprises the sequence of nucleotides according to Sequence ID No. 5. Advantageously, the nucleic acid molecule according to the invention may be used to express the Gas1 protein according to the invention, in a host cell or the like using an appropriate expression vector.

An expression vector according to the invention includes vectors capable of expressing DNA operatively linked to regulatory sequences, such as promoter regions, that are capable of effecting expression of such DNA fragments.

Regulatory elements required for expression include promoter sequences to bind RNA polymerase and transcription initiation sequences for ribosome binding. For example, a bacterial expression vector may include a promoter such as the lac promoter and for transcription initiation the Shine-Dalgarno sequence and the start codon AUG. similarly, a eukaryotic expression vector may include a heterologous or homologous promoter for RNA polymerase II, a downstream polyadenylation signal, the start codon AUG. and a termination codon for detachment of the ribosome. Such vectors may be obtained commercially or assembled from the sequences described by methods well known in the art. Thus, an expression vector refers to a recombinant DNA or RNA construct, such as a plasmid, a phage, recombinant virus or other vector that upon introduction into an appropriate host cell result in expression of the DNA or RNA fragments. Appropriate expression vectors are well known to those skilled in the art and include those that are replicable in eukaryotic cells and/or prokaryotic cells and those that remain episomal or those which integrate into the host cell genome.

The antisense molecule capable of hybridising to the nucleic acid according to the invention may be used as a probe or as a medicament or alternatively in a pharmaceutical composition, by preventing expression of a Gas1 protein. Advantageously, the antisense molecule according to the invention may be used as a drug, or in the preparation of a drug for the treatment of a neurological disorder, such as for example Parkinson's disease, Alzheimer's disease, Huntington's disease, amyotrophic lateral sclerosis, a neurological disorder caused by thrombosis or cerebral trauma. The expression vector including said antisense molecule according to the present invention may be used advantageously *in vivo*, such as in gene therapy (Matteucci & Wagner, Nature 384, Supp 7:20-22 (1996); Whitesell et al. Proc. Natl. Acad. Sci. USA 90:4665-4669 (1993): Wahlestedt, C. Trends Phazmacol. Sci. 15:42-46 (1994)).

Nucleic acid molecules according to the invention may be inserted into the vectors described in an antisense orientation in order to provide for the production of antisense RNA. Antisense RNA or other antisense nucleic acids may be produced by synthetic means.

A further aspect of the invention comprises the host cell transformed, transfected or infected with the expression vector according to the invention, which cell preferably comprises a eukaryotic cell and more preferably a mammalian cell.

Incorporation of cloned DNA into a suitable expression vector for subsequent transformation of a cell and subsequent selection of the transformed cells is well known to those skilled in the art as provided in Sambrook et al (1989) Molecular Cloning, A Laboratory manual, Cold Spring Harbour Laboratory Press.

Nucleic acid molecules according to the invention may also be produced using such recombinant or synthetic means, such as, for example, using PCR cloning mechanisms which generally involve making a pair of primers, which may be from approximately 10 to 50 nucleotides to a region of the gene which is desired to be cloned, bringing the primers into contact with mRNA, cDNA, or genomic DNA from a human cell, performing a polymerase chain reaction under conditions which bring about amplification of the desired region, isolating the amplified region or fragment and recovering the amplified DNA. Generally, such techniques as defined herein are well known in the art, such as described in Sambrook et al (Molecular Cloning: a Laboratory Manual, 1989).

The nucleic acids or oligonucleotides according to the invention may carry a revealing label. Suitable labels include radioisotopes such as $^{32}P$ or $^{35}S$, enzyme labels or other protein labels such as biotin or fluorescent markers. Such labels may be added to the nucleic acids or oligonucleotides of the invention and may be detected using known techniques *per se.*

Further provided by the present invention is a transgenic cell, tissue or non-human organism comprising a transgene capable of expressing the rat Gas1 protein according to the invention. The term "transgene capable of expression" as used herein means any suitable nucleic acid sequence which leads to expression of a Gas1 protein having the same function and/or activity of a rat Gas1 protein according to the invention. The transgene may include, for example, genomic nucleic acid or synthetic nucleic acid including CDNA, integrated into the chromosome or in an extrachromosomal state.

Preferably, the transgene comprises a vector according to the invention, which vector includes a nucleic acid molecule encoding said rat Gas1 protein, or a functional fragment of said nucleic acid molecule. A "functional fragment" of said nucleic acid should be taken to mean a fragment of the gene or cDNA encoding said rat Gas1 or a functional equivalent thereof, which fragment is capable of being expressed to produce a functional rat Gas1 protein according to the invention.

In accordance with the present invention, a defined nucleic acid includes not only the identical nucleic acid but also any minor base variations including in particular, substitutions in bases which result in a synonymous codon (a different codon specifying the same amino acid residue) due to the degenerate code in conservative amino acid substitutions. The term "nucleic acid molecule" also includes the complementary sequence to any single stranded sequence given regarding base variations.

In a further aspect of the invention comprises a protein capable of inducing apoptosis in a cell comprising an amino acid sequence according to Sequence ID No. 4 or a functional equivalent, derivative or bioprecursor thereof.

A "derivative" as defined herein is intended to include a polypeptide in which certain amino acids have been altered or deleted or replaced with other amino acids and which polypeptide retains the biological activity of Gas1 according to the invention and/or which polypeptide can react with antibodies raised using Gas1 according to the invention as the challenging antigen.

Encompassed within the scope of the present invention are hybrid and modified forms of rat Gas1, including fusion proteins and fragments. The hybrid and modified forms include, for example, when certain amino acids have been subjected to some modification or replacement, such as for example, by point mutation yet which modifications still result in a protein which retains the biological activity of Gas1, according to the invention. Specific nucleic acid sequences can be altered by those of skill in the art to produce a protein exhibiting the same or substantially properties to Gas1 of the invention.

A defined protein, polypeptide or amino acid sequence according to the invention includes not only the identical amino acid sequence but isomers thereof in addition to minor amino acid variations from the natural amino acid sequence including conservative amino acid replacements (a replacement by an amino acid that is related in its side chains). Also included are amino acid sequences which vary from the natural amino acid but result in a polypeptide which is immunologically identical or similar to the polypeptide encoded by the naturally occurring sequence.

Proteins or polypeptides according to the invention further include variants of such sequences, including naturally variants which are substantially homologous to said proteins or polypeptides. In this context, substantial homology is regarded as a sequence which has at least 70%, and preferably 80%, 90% or 95% amino acid homology with the proteins or polypeptides encoded by the nucleic acid molecules according to the invention.

Substantial homology should be taken to mean that the nucleotide and amino acid sequences of the Gas1 of the invention display a certain degree of sequence identity. Preferably they share an identity of at least 30%, preferably 40%, more preferably 50%, still more preferably 60%, most preferably 70%, and particularly an identity of at least 80%, preferably more than 90% and still more preferably more than 95 % is desired with respect to the nucleotide or amino acid sequences depicted in Seq. ID Nos. 1 to 4, respectively. A preferred method for determining the best overall match between a query sequence (a sequence of the present invention) and a subject sequence, also referred to as a global sequence alignment, can be determined using, for example, the FASTDB computer program based on the algorithm of Brutlag et al. (Comp. App. Biosci. 6 (1990), 237-245.) In a sequence alignment the query and subject sequences are both DNA sequences. An RNA sequence can be compared by converting U's to T's. The result of said global sequence alignment is in percent identity. Further programs that can be used in order to determine homology/identity are described below and in the examples. The sequences that are homologous to the sequences described above are, for example, variations of said sequences which represent modifications having the same biological function, in particular encoding proteins with the same or substantially the same receptor specificity, i.e. binding specificity. They may be naturally occurring variations, such as sequences from other mammals, or mutations. These mutations may occur naturally or may be obtained by mutagenesis techniques. The allelic variations may be naturally occurring allelic variants as well as synthetically produced or genetically engineered variants.

Antibodies to the Gas1 protein according to the invention may advantageously be prepared by techniques which are well known to those of skill in the art.

The therapeutic or pharmaceutical compositions of the present invention can be administered by any suitable route known in the art including for example intravenous, subcutaneous, intramuscular, transdermal, intrathecal or intracerebral or administration to cells in *ex vivo* treatment protocols. Administration can be either rapid as by injection or over a period of time as by slow infusion or administration of slow release formulation. For treating tissues in the central nervous system, administration can be by injection or infusion into the cerebrospinal fluid (CSF).

Gas1 protein, the antisense molecules or indeed the compounds identified as enhancers or inhibitors of activity or expression of Gas1 or the otherwise lethal proteins may be used in the form of a pharmaceutical composition, which may be prepared according to procedures well known in the art. Preferred compositions include a pharmaceutically acceptable vehicle or diluent or excipient, such as for example, a physiological saline solution. Other pharmaceutically acceptable carriers including other non-toxic salts, sterile water or the like may also be used. A suitable buffer may also be present allowing the compositions to be lyophilized and stored in sterile conditions prior to reconstitution by the addition of sterile water for subsequent administration. Incorporation of the aforementioned compounds or antisense molecules, for example, into a solid or semi-solid biologically compatible matrix may be carried out which can be implanted into tissues requiring treatment.

The carrier can also contain other pharmaceutically acceptable excipients for modifying other conditions such as pH, osmolarity, viscosity, sterility, lipophilicity, solubility or the like.

Pharmaceutically acceptable excipients which permit sustained or delayed release following administration may also be included.

The compounds identified in accordance with the method of the invention may be administered orally. In this embodiment they may be encapsulated and combined with suitable carriers in solid dosage forms which would be well known to those skilled in the art.

As would be well known to those of skill in the art, the specific dosage regime may be calculated according to the body surface area of the patient or the volume of body space to be occupied, dependent upon the particular route of administration to be used. The amount of the composition actually administered will, however, be determined by a medical practitioner, based on the circumstances pertaining to the disorder to be treated, such as the severity of the symptoms, the composition to be administered, the age, weight, and response of the individual patient and the chosen route of administration.

The invention may be more clearly understood from the following examples and accompanying figures wherein:

Figure 1 is a graphic representation of induction of cell death in hippocampal neurons by transient overexpression of Gas1. Quantitative analysis of the effect of rat Gas1 overexpression is represented as % survival 24 hours after transfection of the different expression vectors and the combination of expression vectors, that is showed under each column. Values are referred to 100% survival which corresponds to transfection with empty vector pcDNA3 as indicated in the first column. Overexpression of Gas1 resulted in a reduction of neuronal survival to 30% (1.5 µg of Gas1, rg1) or 50% (0.75 µg of Gas1, rg1*). Overexpression of the C-terminal truncated form of Gas1 (ΔC) did not have a significant effect on cell survival at 24 hr. Cotransfection of Gas1 and Bcl-2 resulted in the total protection of the neurons (rgl*+Bcl-2*). While overexpression of Bcl-2 alone (1.5 µg) or the complementary form of Gas1 (AS, 1.5 µg) did not result in survival values different from the control. Results are expressed as mean ± SEM. Statistical analysis was performed by Student's t-test. *** denotes $p>0.001$, and other treatment groups were statistically non-significant ($p<0.5$).

Figure 2 is a graphic representation of the effects of protection against cell death by an antisense Gas1 oligonucleotide and by complementary Gas1 RNA.

A.- Antisense Gas1 (25 µM Ag1 blocks NMDA-induced neuronal death in cortico-hippocampal cultures. The viability of mature cortico-hippocampal cultures was analyzed 24 hr after 500 mM NMDA exposure. Pretreatment with the different antisense oligonucleotides at the indicated concentrations was done 24 hr before the exposure to NMDA. Results are expressed as mean ± SEM. Statistical analysis was performed by Student's t-test. *** denotes $p>0.001$, and other treatment groups were statistically non-significant ($p<0.5$).

B.- Antisense Gas1 (25 µM Ag1) prevents the reduction in survival induced by 200nM staurosporine in NB69 wild type cells (NB69wt). Columns 1 and 2. Moreover, stable expression of complementary Gas1 RNA in NB69-Gas1 cells protects from staurosporine-induced neuronal death as compared to NB69 mock cells stably transfected with the empty vector. Columns 3 and 4. The viability was analyzed 24 hr after exposure to staurosporine (200nM) in all the cases. Results are expressed as mean ± SEM. Statistical analysis was performed by Student's t-test. *** denotes $p>0.001$, and other treatment groups were statistically non-significant ($p<0.5$).

Figure 3 is an illustration showing increase in LDH levels in the culture media at different times after doxicycline removal from indicated clones.

Figure 4 is an illustration of the results obtained form a Western blot analysis showing the induction of Gas-1 immunoreactivity after doxicycline removal from indicated clones.

Figure 5 is an illustration of the results obtained from staining with Annexin-V Fluos (green) before (A) and 24 h. after (B) removal of doxicycline in cultures of NB69-MC3 cells. Propidium iodine staining could be observed at later times (48 h. or more) after doxicycline removal © and D).

Figure 6 is a diagrammatic representation of results showing reduced LDH release to the culture media after doxicycline removal in C3 cells transiently transfected with IAP.

Figure 7 is a diagrammatic representation of results showing increase in LDH levels in the culture media and reduced beta-galactosidase activity after transient transfection of wtGas1 or mutants Δ1 to Δ3.

Figure 8 is an illustration of results showing fragmentation of chromosomal DNA in NB69 cells treated with 200 nM staurosporine is prevented by stable overexpression of antisenseGasl. Standard ladder DNA is shown in lane 1 (M).

Figure 9 is a diagrammatic representation of the increase in LDH release and the reduction in beta-galactoside activity after overexpression of human mGluR1 in NB69 cells is blocked by cotransfection of antisense Gas1.

**Example 1.- Cloning rat Gas1**

50,000 primary phages from a CDNA library prepared from cortico-hippocampal primary cultures 6 hr after a brief (5 min.) exposure to 500 mM of NMDA were differentially screened. Primary cortico-hipocampal cultures were prepared from E17 rat fetuses essentially as described (Choi, 1987). Neuronal cultures were used for the experiments after twelve days in culture. To prepare the libraries, poly-A+ mRNA was prepared from cortico-hippocampal control or NMDA treated cultures using the microFastTrack kit from Invitrogen. Oligo-dT primed cDNA was cloned in 1ZAP (Stratagene) following manufacturer's instructions. Fifty thousand phages were differentially screened, using $^{32}$P-labeled cRNA probes derived from poly-A+ RNA isolated from control cultures (- probe), or from cultures 6 hr after the NMDA treatment (+ probe) and the use of the AMV reverse transcriptase (25 U, BRL). For differential screening of the library prepared from treated cultures, each pair of nitrocellulose filters upon which phages have been transferred are subjected to probe (+) or probe (-) hybridization for 20 hours at 42° C in a hybridization buffer that contains 50% formamide, 10% dextrane sulfate, 4xSSC, 0.1% SDS, Tris-HCl 10 mM, pH 7.4, lxdenhardt's, 50 (g/ml) and salmon sperm and the corresponding probe 106 dpm/ml. Then the filters are washed for 30 min. at room temperature in 2xSSC, 0.1% SDS, twice for 30 min. At 42° C in 2xSSC, 0.1% SDS and twice for 30 min. At 55° C in 0.2xSSC, 0.1% SDS. After autoradiographic exposure for five days, the hybridization signals obtained for each pair of filters hybridized with probe (+) or probe (-) are compared, looking for the differential presence of hybridization signal in filters (+) in relation to (-). In this way a 714 bp clone initially named pCHN-414 is obtained, and once sequenced, it showed homology with the untranslated 3' region of the Gas1 human and rat gene. This indicates that the pCHN-414 clone corresponds to the rat Gas1 gene, which is confirmed in the following experiments.

A cDNA fragment containing the complete coding sequence of rat Gas1 was obtained following PCR using rat genomic DNA as template. The 5' primer was derived from the mouse sequence (Del Sal et al., 1992) at position 49-66 (5'-GAATTCGAGAAACGCTCCGAGTTTCG-3'). The sequence of the 3' primer was obtained from the rat clone (5'-GGATCCAGTTTTAATACAGTTTATACGACGTACCAGG -3'), at a position corresponding to 2449-2483 in the mouse sequence. Flanking EcoRI and BamHI sites in the 5' ends of the oligonucleotides were designated for cloning purposes. The DNA was then amplified by the polymerase chain reaction using Tli DNA polymerase (Promega) for 40 cycles. The timing for each cycle was as follows: 1 min. at 94°C, 1 min. at 60°C and 2 min. at 72°C. The cycling was preceded by 2 min. denaturing period at 94°C and followed by a 7 min. extension at 72°C. A 2.4 kb PCR product was obtained, restricted by EcoRI, cloned into pCDNA3 (Invitrogene) and completely sequenced at both strands using Thermo stable Sequenase and the conditions suggested by the manufacturer (Amersham-Pharmacia).

**Example 2.- Gas1 gene transfection induces neuronal death**

To assess the effect of the Gas1 protein on neuronal viability, pGas1 was cotransfected together with a β-galactosidase expression vector that served as a marker of transfected neurons using liposomes. For optimal liposome-mediated transfection efficiency these experiments were performed in serum-free conditions using primary cultures of hippocampal neurons. Culture of hippocampal neurons was performed in chemically defined medium as described (Ohsawa, et al, 1993. Response of embryonic hippocampal neurons in culture to neurotrophin-3, brain-derived neurotrophic factor and basic fibroblast growth factor. Neurosci. 57, 67-77.). Hippocampal neurons (3 DIV, 2 x $10^6$ cells per 35 mm dish) received 2 μg total amount of plasmid DNA containing 0.5 μg of β-galactosidase expression vector (pCH110, Pharmacia) and 1.5 μg of pCDNA3 vector (InVitrogene), empty or containing the Gas1 coding sequence in the 5' to 3' orientation (rg1), the Gas1 coding sequence in the complementary orientation (3' to 5', AS) or the truncated Gas1 (ΔC, The C-terminal truncated expression vector for Gas1 contains amino acids 1 to 229 and was prepared from the rg1 plasmid by subcloning of a HindIII/EcoRI fragment into pcDNA3). For the protection by Bcl-2, 0.75 μg of rg1 and 0.75 μg of pBcl-2 were used together with 0.5 μg of pCH110. A ratio of 1:2 DNA: Transfectam (Promega) was used for the preparation of liposomes. Three hours after transfection, the liposome-containing medium was replaced with fresh medium. To assess the number of transfected neurons, the cultures were fixed with 2.5% glutaraldehyde 24 hr after the transfection and X-Gal staining was performed as suggested (Promega). The experiments were repeated 3 to 5 times, performed in quadruplicates and each plate was counted by three independent investigators. X-Gal positive neurons present in 20 fields of each plate were counted and averaged. The average of X-Gal positive neurons in control cultures was taken, as 100% survival for each experiment. The transfection efficiency was similar for the different plasmids, for different preparations of a given plasmid and among experiments, as assayed following a similar protocol but 16 hr after transfection, a time point at which cell death has not started. The transfection efficiency accounted for approximately 1 % of the total cell population, and showed little variation between the different experimental groups and between experiments.

At 24 hr after transfection, the total number of X-Gal positive neurons in cultures transfected with rg1 was dramatically reduced. The survival after Gas1 overexpression was decreased in a concentration-dependent manner to 30% and 50% when transfected with 1.5 and 0.75 µg of pGas1, respectively (Fig. 1) . In contrast, the overexpression of the complementary strand of Gas1 (AS), did not affect the number of viable X-Gal positive cells at 24 hr after transfection (Fig. 1). These results associate the overexpression of Gas1 with neuronal death and indicate that high levels of Gas1 protein are sufficient to trigger a death process that is accompanied by profound alterations in morphology.

**Example 3.- Blocking of translation of the Gas1 protein by Gas1 antisense oligonucleotide or by Gas1 complementary chain overexpression protects against excitotoxic death or death induced by staurosporine.**

NMDA-induced excitotoxicity was analyzed in cortico-hippocampal cultures maintained for 12-14 DIV similar to those already described in Example 1. To apply the excitotoxic insult, the cultures were rinsed twice with Locke's solution without Mg2+ and exposed to 500 mM NMDA (Sigma) for 5 min. in the same Locke's solution or to Locke's solution alone as control. NMDA was washed out, the Mg2+ concentration was restored by 2 changes of Locke's +Mg2+ solution, and the original medium was replaced. Phase-bright bipolar cells, taken to represent living neurons, were counted 24 hr after the NMDA exposure. Alternatively, the vital staining method (Jones and Senft, 1985 An improved method to determine cell viability by simultaneous staining with fluorescein diacetate-propidium iodide. J Histochem Cytochem 33:77-79) was used to assess the extent of neuronal death and the protection by antisense oligonucleotide treatments. Similar results were obtained using either method. The vital staining method was used to prepare Fig. 2a.

A 15-mer Gas1 antisense oligonucleotide 5'-TCCTCATCCATCCAT-3', (AG1) spanning the start site of translation (underlined) was used to specifically block Gas1 translation. As a negative control a 15-mer oligonucleotide with nucleotide substitutions 5'-TCCTCATCGATGGTA-3' (AG1mut) was used and the Gas1-unrelated antisense oligonucleotides for cyclin D1: 5'- GAGCTGGTGTTCCAT -3'(Matsushime et al., 1991 Colony-stimulating factor 1 regulates novel cyclins during the G1 phase of the cell cycle Cell 65:701-713) and for zif268: 5'- GTAGTTGTCCATGGT -3' (Milbrandt, 1987 A nerve growth factor-induced gene encodes a possible transcriptional regulatory factor. Science 238:797-799). All oligonucleotides were protected at each end by two phosphorothioate groups. The oligonucleotides were added to the culture medium 24 hr before the induction of neuronal death by NMDA, at the concentrations indicated in the figures. Experiments were done in triplicate and repeated as least five times.

Staurosporine-induced neuronal death was assayed in human neuroblastoma NB69 cells cultured in DMEM/HAM F12 medium supplemented with 10% fetal calf serum, 2 mM glutamine and 50 µg/ml gentamycin. Wild-type or stably transfected NB69 cells (2 x 104 cells/35 mm dish) were exposed to 100 nM staurosporine (RBI). Phase-bright cells without signs of membrane or neurite degeneration were counted 24 hr later. Experiments were done in triplicate and repeated as least five times. Stably transfected NB69 cells that overexpress Gas1 in the antisense orientation (NB69-Gas1 were prepared by the calcium phosphate precipitation method followed by two weeks of geneticin selection. Control cells were prepared in parallel by transfection with pCDNA3 vector alone (NB69-vector). Expression of the constructs in the cell clones were analyzed by northern blot analysis, and the clones having the highest expression were chosen for the experiments. Stable tranfectants were kept in the presence of 0.3 mg/ml of geneticin, which was removed before plating for the experiments.

Neuron death by overstimulation of excitatory amino acid receptors is a well characterized phenomenon that receives the generic denomination of excitotoxicity. Excitotoxicity is a complex process that involves significant changes in the cell cycle gene expression.

The detection of various of these genes was carried out in the course of differential screening of primary culture rat cortico-hippocampal neuron library at an excitotoxic concentration (500 mM) of N-methyl D-aspartic-NMDA acid (Choi, 1987, Ionic dependence of glutamate neurotoxicity in cortical cell culture. J. Neurosci 7:369-379). Cloning and sequence determination of one of them identified it as part of the rat homologue of the Gas1 gene described above in humans. Subsequently, the complete rat Gas1 gene was cloned and sequenced, as referred to below whose nucleotide sequence is attached as SEQ ID 1, obtaining from it the amino acid sequence of the rat Gas1 protein whose sequence is attached as SEQ ID 2.

Subsequently, the increase of 8 to 10 times the Gas1 mRNA expression after 6 hours of administering a pulse of NMDA in primary cortico-hippocampal cell cultures in contrast to control cells was confirmed by Northern blot. On the other hand, Gas1 mRNA induction has not been observed in pure primary culture of hippocampal astroglia after exposure to NMDA or glutamate (data not shown), suggesting that Gas1 overexpression occurs in neurons as part of the gene response during NMDA induced neuronal degeneration.

Hence, Gas1 expression in experimental models where neuron death was induced by means of different harmful stimuli has been evaluated. First of all, intraperitoneal administration of kainic acid was used, an experimental model that has demonstrated that it produces acute and delayed neuron death in different cerebral areas that include the hippocampus, olfactory cortex, thalamus and amygdala ((Schwob et al, Widespread patterns of neuronal damage following systemic or intracerebral injections of kainic acid: a histological study. Neurosci 5:991-1014; 1980; Sperk et al., Kainic acid-induced seizures: neurochemical and histopatological changes. Neurosci 10:1301-1315;1983). Northern blot analysis demonstrated that Gas1 expression in control rat brain (without administering kainic acid) was very low in accordance with previous data (Del Sat et al., 1994). However, strong induction of Gas1 expression in hippocampus and olfactory cortex after intraperitoneal administration of 10 mg/kg of kainic acid was observed. Already detectable induced Gas1 mRNA levels after kainic acid appear 6 hours after the beginning of treatment and were maintained for at least 10 days. In order to reveal Gas1 induction and to confirm neurone localization thereof in situ hybridization after administering kainic acid was performed. A RNA specific for Gas1 monocatenary probe revealed, 24 hours after administration of kainic acid, signs of intense hybridization in isolated neurons, being evident in the pyramidal layer of the CA1 area of the hippocampus. Similar results were observed after provoking an experimental ischemic process in gerbil brain by transient occlusion of both carotids, giving rise to rapid degeneration of a numerous population of pyramidal neurons of the CA1 layer of the hippocampus. It was observed that this massive neuron death was preceded by a significant rise of Gas1 mRNA and Gas1 protein levels (evaluated by immunocytochemistry) in those neurons. Likewise, it was also observed that neuron loss in all the cerebral cortex that is produced in rats in the perinatal period after exposure to ionizing radiation is preceded by high induction of Gas1 mRNA. All these results suggest that Gas1 is involved in regulation of neuron death by excitotoxicity.

In a subsequent step to show a direct relationship between Gas1 gene expression and neuron death, hippocampal neurons were transfected with the pcDNA3 eukaryotic expression vector containing the Gas1 (rg1) gene. The experiments were carried out in primary cultures of rat fetus hippocampal neurons that were transfected with different amounts of the Gas1 expression vector, evaluating a posteriori the survival of these cells (Fig. 1). Hence, it was observed that transfection of cells with 1.5 μg of rg1 construct caused a significant reduction of survival, obtaining levels of survival lower than 40%, with regard to the control group (transfected with the empty expression vector), or with regard to the group of cells transfected with the Gas1 (SEQ ID 3) complementary or antisense sequence (Antisense (AS) column, see figure 1). The effect depends on the amount of Gas1 construct transfected and thus when transfection took place with half the amount (rg1*) the reduction of survival was more limited although still significant (Fig. 1).

On the other hand, this reduction of survival of neurons transfected with Gas1 (rg1* column) is completely blocked by coexpression in these same neurons with the Bcl-2 protein (rg1*+Bcl-2 column), Bcl-2 being the antiapoptotic protein prototype (Fig. 1). On the other hand, these experiments with rat Gas1 were repeated in NB69 human cells and in NIH3T3 rat fibroblasts, obtaining similar results. Besides, NB69 human neuroblastoma and 3T3 rat fibroblast cells were transfected with human Gas1, observing cell death of the NB69 and 3T3 cells.

Initially, Gas1 expression has been related to the G0 phase of cell cycle arrest in rat fibroblasts deprived of serum (Del Sal et al., 1992). However, the present invention shows that Gas1 overexpression in cortico-hippocampal neurons induces neuron death, acting as a protein with apoptosis inductive activity.

Up to now it had been described that Gas1 protein was a cell membrane protein and as of its amino acid sequence the presence of two transmembrane segments had been proposed and a hypothetical RGD domain that may interact with integrin, the only domain described that relates its structure to its arrest function of the cell cycle (Schneider et al.). The present inventors have succeeded in overexpressing the Gas1 protein by means of a prokaryotic vector (pTrcHis) in bacteria for subsequent purification in sufficient amounts to carry out protein studies. Hence, it was observed in experiments of artificial bilayers that the purified Gas1 protein is capable of inserting in and forming cationic channels and that said capacity may be linked to neuron death induction. Aside from the already described location of this protein in the cell membrane we have located the presence of Gas1 in the perinuclear and mitochondrial membrane. It must be pointed out that Bcl-2 and Bax, proteins related to the cell cycle, are also located in the mitochondrial membrane and are capable of forming ionic channels. This common location of these proteins makes compatible a possible functional interaction of Gas1 with Bax type proapoptotic proteins and Bcl-2 and Bcl XL antiapoptotic proteins. The present inventors have also been able to demonstrate, by mutational analysis of the RGD domain, that this is not implied in neuron apoptotic activity of the Gas1 protein. On the contrary, we have delimited the neuron death inductive capacity in an amphipathic domain in the Gas1 terminal carboxyl region, located between the amino acids 174 and 304 of the protein (SEQ ID 4), that does not coincide with any of the previously hypothesized domains.

In order to confirm the relevance of this region of the protein in the lethal capacity of Gas1 hippocampal neurons were transfected with a mutated Gas1 construction in that region (ΔC column, Fig. 1). As seen in Fig. 1, survival of these neurons is similar to that verified in the control group (vector column) and is significantly higher than that of neurons transfected with wild type Gas1 (rg1 and rg1* columns).

Thanks to the exact knowledge of the structure activity relationship in Gas1 polypeptide there is now a rational base to understand the lethal action of Gas1 as well as for the development of molecules capable of blocking the Gas1 polypeptide function or Gas1 gene expression with which to create new therapeutic tools to reduce or eliminate cell death. For this purpose, overexpression of Gas1 protein in human neuroblastoma cells or in rat fetus neurons by means of transfection with liposomes of the Gas1 eukaryotic expression vector was used. This may be constitive expression (pcDNA3) in transient expression but it may also be an inducible expression (pIND) that permits preparation of stable lines that will express Gas1 after induction of the expression vector for example with muristerone in the case of pIND. To evaluate the phenomenon of death one can resort to morphological criteria by means of transfection with b-galactosidase or by means of GFP (green fluorescent protein) or to biochemical criteria of degradation, detection of nucleic acids or release of dehydrogenase lactate into the medium, or any other indication of cellular lysis. The specific application of this invention is its use as a screening system of molecule collections in the search for products active in blocking Gas1 polypeptide function or Gas1 gene expression and therefore, providing a protective action against cell death. These screening systems form part of the present invention.

Transgenic models provide a useful model for assaying and testing drugs for their effectiveness and safety in the treatment of the above described diseases. Also, by means of the use of specific tissue promoters or cellular phenotype promoters Gas1 expression can be achieved in target tissues and cells for a better and more specific testing. An advantage of the invention is that potential Gas1 inhibitors and/or blocking agents can be readily tested in an *in vivo* model that closely mimics a human by the use of a rat gene for Gas1.

Furthermore, a specific Gas1 antisense oligonucleotide (SEQ ID 5) was verified as being able to block the translation of Gas1 protein and this involves a total blocking of the NMDA induced neuron death phenomenon in primary neuron cultures (survival practically of 100%, 25mM Ag1 column in Fig. 2a) or by staurosporin induced death (200nM) (Fig. 2b).

Upon observing that the stable expression of high levels of Gas1 complementary messenger in the NB69-Gas1 cell lines makes these cells more resistant to external harmful stimuli, it was possible to use these cells for forced expression of lethal genes that would induce cell death in cells but that would not cause death in these Gas1 expressing protected lines. An example of the possible application of this protected system (NB69-Gas1) is the overexpression of the glutamate type I metabotropic receptor (mGluR-I).

This provides a system which permits the study of the pharmacology of mGluR-I and to screen possible agonist or antagonist molecules of this receptor. The example presented for mGluR-I is only one of the multiple possibilities of using Gas1 inhibition to achieve stable expression systems for genes that are normally detrimental to the survival of a cell. Other lethal proteins to evaluate by means of this expression system comprise, for example, ionotropic glutamate receptors including NMDA-receptors, AMPA receptors, kainate receptors with their various subunits and variants: metabotropic glutamate receptors (including subtype 1 to 8); other excitatory amino acid receptors (e.g. taurine); cytokine receptors; chemokine receptors; mono amine receptors; peptide receptors; enzymes such as kinases, caspases; and any other protein in signal transduction cascade mediating cell death.

The present inventors have also developed and produced polyclonal rabbit antibodies and rat hybridomas which can produce Gas1 monoclonal antibodies. These antibodies have been used in the immunocytochemical and Western blot studies that are described in this invention for the purpose of evaluating the expression of Gas1 protein. It is to be emphasized that these antibodies may be used for analytical purposes to purify said polypeptide, for in vitro diagnosis or for therapeutic purposes of disorders, especially those that imply cell death and neurodegeneration, where this polypeptide is expressed.

**Effect of functional inhibition of Gas1 on neuronal viability.**

**Preparation of a human Gas1 inducible expression vector using the Tet-Off system.** Firstly, an attempt was made to obtain double transfectants RXR-pINDhGas1 for ecdysone-induced expression of hGas-1 in the human neuroblastoma cell line NB69. This approach proved to be difficult and no clones showing a reasonable growth rate could be selected. In most cases, double transfectants presented long neuritic processes corresponding to a phenotype of terminally differentiated neurons, had insignificant incorporation of $^{3}$H-thymidine and a notorious accumulation of apoptotic bodies could be observed in the cultures. Attempts to reduce the percentage of serum in the media or to culture under serum-free conditions did not significantly improve the situation. It may be that a very high expression of the RXR receptor in NB69 primary clones NB69-RXR4 and NB69-RXR7 leads to a ligand-independent activation of the pINDhGas1 vector which could be the origin of the problem. Accordingly, NB69-RXR clones expressing low or moderate levels of the RXR receptor were tried. Thus, NB69 cells were cotransfected with RXR and pINDhGas1 and a double selection with G418 and Zeocin was performed. Three weeks after transfection clones could be observed. However, the growth rate of these clones was again very slow and abundant apoptotic bodies could be distinguished surrounding and on the top of each clone. In view of these negative results the ecdysone-based approach was abandoned in favour of a different inducible system performed in this case the Tet-Off system from (available from Clontech). Two complete rounds of selection were performed. NB69 cells were initially stably transfected with the TetOff plasmid using G418 for selection. Twelve clones were chosen in each round, 1 to 12 in the first and A to L in the second, for checking of expression levels of the TetOff repressor by transient transfection with reporter TRE-Luc again available from Clontech. After this analysis, clones 3, 8 and C were finally selected for cotransfection with the inducible expression vector TRE-hGas1 (a plasmid including the hGas1 sequence cloned in the pTRE2 vector available from Clontech) and the selection vector TK-hyg (Clontech). Selection was carried out for two weeks using 2 mg/ml of hygromycine in the presence of 1 µg/ml of doxicycline to keep the repressor TetOff bound to the TRE promoter in the TRE-hGas1 construct. Twelve subclones were again chosen from each of the three groups (3, 8 and C) and Gas1-induced cell death was assayed after doxicycline removal using the release of lactate dehydrogenase (LDH) as an index of cell damage (Cytotox kit, Promega). Positive clones were further analyzed for the inducibility of Gas1 after doxicycline removal by western blot using the monoclonal antibody G8F9-1 specific for Gas1. Some of these results are summarized in figures 3 and 4. As a result, five TetOff-inducible clones were finally selected: 8d, 8e, 8f, C2 and C3.

**Analysis of the molecular mechanism of Gas1-induced cell death.** To assess the cell death mechanism after induction of Gas1 by doxicycline removal we first processed each of the clones using staining for annexin-V (Annexin-V Fluos, kit, Roche) as a hallmark of apoptosis and the staining with propidium iodine as an indication of a necrotic mechanism. As shown in figure 5 using clone C3, an intense green fluorescence in the outer membrane was observed soon after doxicycline removal, while at later times some of the cells developed an intense nuclear red fluorescence due to the entrance of the intercalating agent propidium iodine (Fig. 5C and D). These results indicate that the mechanism of death involves a purely apoptotic process, at least at the early stages. Because of this the effect of caspase inhibition on Gas1-induced cell death after doxicycline removal was tested. For that, clones C2 and C3 were transfected with different expression vectors for crmA, IAP and p35, three caspase inhibitors with distinct specificity for different caspases. In these experiments it was found that overexpression of IAP, a caspase inhibitor of non-viral origin, selectively blocked Gas1-induced cell death after doxicycline removal (Fig. 6). This result indicates that inhibition of the pro-caspase 9 or the effector caspases 3, 6 and 7 are involved in the death process triggered by Gas1. Further studies using more selective caspase inhibitors as well as specific substrates for the different caspases are needed to identify the caspase directly responsible of the death after Gas-1 induction.

**Molecular analysis of death-related domains in Gas1.**

**Domain analysis of the amphipathic α-helix region of Gas1.** As a result of the frame-shift strategy it was possible to delineate a domain encompassing amino acids 174 to 279 in rat Gas1 involved both in the channel activity as well as in the death-inducing properties of Gas1. To more precisely map the specific residues responsible for these two biological activities deletion of discrete fragments of Gas1 within the 174-279 region was performed. Three deletion mutants have, so far, been prepared and analyzed Gas1 Δ1 to Δ3. Transient cotransfection experiments of each construct with an expression vector for lacZ was performed in NB69 cells and release of LDH and beta-galactosidase activity was measured as an index of cell death and viability, respectively. In all cases, a lethal effect similar to wtGasl was observed. The results of a representative experiment are shown in figure 7.

**Effect of knockout of Gas1 on neuronal viability.**

**Stable expression of antisense Gas1 RNA in NB69 cells: effects on survival.** To develop a cellular model highly resistant to the overexpression of potentially toxic proteins i.e. metabotropic glutamate receptors, we selected NB69 clones stably transfected with an expression vector for Gas1 cloned in the antisense orientation. The initial screening of positive clones was performed by Northern blot. The clone showing higher levels of antiGas1 mRNA was then tested for its resistance to neuronal death induced by staurosporine. In previous it was observed that staurosporine-induced neuronal death involves the induction of endogenous Gas1 and could be prevented by administration to the culture media of the antisense oligonucleotides described herein specific for the amino-terminal of Gas1. As shown in figure 8, the DNA laddering associated with the apoptosis induced by staurosporine was not observed in cells stably transfected with antisense Gas1.

**Stable expression of human mGluR1 in NB69 cells: protection by antisense Gas1.** The suitability of our NB69-ASGas1 cells to support the stable overexpression of potentially lethal proteins was then tested. In pilot experiments, transient transfections in NB69 cells with an expression vector for the human metabotropic GluR1 was performed and the effects on viability after cotransfection with antisenseGas1 expression vector were compared. The results were again evaluated using the two independent tests of cell death; release of lactate dehydrogenase to the culture media at different times and the survival of transfected cells 48 hours after transfection using the expression of LacZ as an interval reporter. The results obtained from a typical experiment performed in triplicate are shown in Figure 9.

**Claims**

**1.** An in vitro method of inhibiting the lethal effect of expressing an otherwise lethal protein in a neuronal cell, wherein said protein normally induces the expression or activates either the Gas1 protein or a protein in the signal transduction pathway of which Gas1 is a component, said method comprising:

(a) using a neuronal cell,or a neuronal tissue having (i) a nucleotide sequence encoding a Gas1 protein, or a Gas1 derivative, which is capable of inducing apoptosis in said cell and (ii) a further nucleotide sequence encoding said otherwise letal protein
(b) inhibiting function and/or expression of said Gas1 protein or Gas1 derivative; and
(c) expressing said sequence encoding said otherwise lethal protein.

**2.** An in vitro method of identifying compounds which inhibit or enhance expression or activity of proteins which are lethal to a neuronal cell, wherein said proteins normally induce the expression or activates either the Gas1 protein or a protein in the signal transduction pathway of which Gas1 is a component, said method comprising:

(a) using a neuronal cell or neuronal tissue comprising a nucleotide sequence encoding a Gas1 protein or a Gas1 derivative, which is capable of inducing apoptosis in said cell, and ii) a further sequence encoding said otherwise letal protein;
(b) inhibiting function and/or expression of said Gas1 protein or Gas1 derivative or a protein in the apoptotic pathway of which Gas1 is a component;
(c) expressing said sequence encoding said otherwise lethal protein;
(d) contacting said cell with a compound to be tested; and
(e) monitoring the effect of said compound on said otherwise lethal protein compared to an identical cell which has not been contacted with said compound.

**3.** A method according to claim 1 or 2 wherein said expression or activity of Gas1 protein is inhibited by providing a nucleic acid molecule in said cell which is capable of hybridising to mRNA corresponding to Gas1 DNA to prevent expression thereof.

**4.** A method according to claim 1 or 2 wherein said expression or activity of said Gas1 protein is inhibited by inhibiting the expression or activity of a protein in the pathway of which Gas1 is a component.

**5.** A method according to any of claims 1 to 4 wherein said cell is induced to express said Gas1 protein by transfecting said cell with an inducible expression vector encoding a Gas1 protein or Gas1 a derivative which is capable of inducing apoptosis in a neuronal cell.

**6.** A method according to claim 5 wherein said cell is induced to express said Gas1 protein by contacting said cell with an inducing agent, such as muristerone.

**7.** A method according to any of claims 1 to 6 wherein said further sequence encoding said otherwise lethal protein is expressed by providing it on a suitable expression vector.

**8.** A method according to any of claims 1 to 7 wherein said lethal protein is a highly expressed recombinant protein.

**9.** A method according to any of claims 1 to 7 wherein said otherwise lethal protein comprises any of a glutamate, NMDA, AMPA or kainate receptor.

**10.** A method according to claim 9 wherein said glutamate receptors comprises any of a type 1 to 8 metabotropic receptor.

**11.** A method according to any of claims 3 to 9 wherein said nucleic acid molecule is provided as an oligonucleotide or as a vector including a nucleotide sequence of said nucleic acid molecule.

**12.** A method according to claim 11 wherein said nucleic acid molecule comprises an oligonucleotide consisting of the nucleotide sequence depicted in Sequence ID No. 5.

**13.** A method according to claim 11 wherein said nucleic acid molecule further comprises ribozyme or DNAzyme activity.

**14.** A method according to any of claims 1 to 13 wherein said Gas1 protein is of mammalian origin.

**15.** A method according to claim 14 wherein said Gas1 protein is from any of a human, mouse or rat.

**16.** A method according to claim 14 or 15 wherein said Gas1 protein comprises the amino acid sequence depicted in either of Sequence ID No. 2 or 4 or a Gas1 derivative which is capable of inducing apoptosis in a neuronal cell.

**17.** A nucleic acid molecule encoding a protein capable of inducing apoptosis in a neuronal cell comprising an amino acid sequence according to Sequence ID No. 4 or a nucleic acid molecule complementary thereto.

**18.** A nucleic acid molecule according to claim 17 which is a DNA sequence.

**19.** A nucleic acid molecule according to claim 18 which is a cDNA molecule.

**20.** A nucleic acid molecule according to claim 17, 18 or 19 comprising the sequence of nucleotides according to Sequence ID No. 1.

**21.** Use of an antisense molecule, capable of hybridising to the nucleic acid molecule of any of claims 17 to 20 under conditions of high stringency, in a method according to any of claims 1 to 16.

**22.** Use of an antisense molecule according to claim 21 comprising a sequence of nucleotides according to Sequence ID No. 3 or 5.

**23.** A protein capable of inducing apoptosis in a neuronal cell comprising an amino acid sequence according to Sequence ID No. 4.

**24.** An expression vector comprising a nucleic acid molecule according to any of claims 17 to 20.

**25.** An expression vector according to claim 24 wherein said vector is any of a plasmid, virus or phage derived vector.

**26.** An expression vector according to claim 24 or 25 comprising a tissue or cell specific promoter.

**27.** An expression vector according to any of claims 24 to 26 further comprising a sequence encoding a proapoptotic protein.

**28.** An expression vector according to any of claims 24 to 27 which is inducible for expression of said Gas1 polypeptide or said polypeptide capable of inducing apoptosis in a cell.

**29.** An expression vector according to claim 28 comprising the inducible vector pIND.

**30.** A host cell, tissue or non-human organism, transformed, transfected or infected with a vector according to any of claims 24 to 29.

**31.** An in vitro method of identifying compounds capable of preventing or accelerating Gas1 mediated cell death comprising the steps of:

(a) contacting a neuronal cell, expressing Gas1 or a Gas1 derivative capable of inducing apoptosis in a cell with said compound to be tested; and
(b) monitoring the effect of said compound on the state of said cell compared to a neuronal cell which has not been contacted with said compound.

**32.** A method according to claim 31 wherein said cell in step (a) comprises a cell according to claim 30.

**33.** A pharmaceutical composition comprising an antisense molecule according to claim 21 or 22, together with a pharmaceutically acceptable carrier, diluent or excipient therefor.

**34.** Use of an antisense molecule according to claim 21 or 22, in the manufacture of a medicament for the prevention or treatment of a neurological disorder mediated at least in part by expression of a Gas1 protein or a derivative thereof capable of inducing apoptosis in a cell or a protein in the pathway of which Gas1 is a component.

**35.** Use according to claim 34, wherein said neurological disorder is any of, Parkinson's disease, Alzheimer's disease, Huntington's disease, amyotrophic lateral sclerosis, a neurological condition caused by thrombosis or cerebral trauma.

**36.** Use of an antibody capable of binding to a protein according to any of claims 28 to 30 in a method according to claims 2 or 31.

**Patentansprüche**

**1.** In-vitro-Verfahren zur Hemmung des letalen Effekts der Expression eines anderenfalls letalen Proteins in einer neuronalen Zelle, wobei das Protein normalerweise die Expression induziert oder entweder das Gas1-Protein oder ein Protein in dem Signaltransduktionsweg, zu dem Gas1 als Bestandteil gehört, aktiviert, wobei das Verfahren:

a. die Verwendung einer neuronalen Zelle oder eines neuronalen Gewebes, die bzw. das (i) eine für ein Gas1-Protein oder ein Gas1-Derivat, das zur Apoptoseinduktion in der Zelle fähig ist, codierende Nukleotidsequenz und (ii) eine weitere, für das anderenfalls letale Protein codierende Nukleotidsequenz aufweist,
b. die Hemmung der Funktion und/oder der Expression des Gas1-Proteins oder Gas1-Derivats sowie
c. die Expression der für das anderenfalls letale Protein codierenden Sequenz umfaßt.

**2.** In-vitro-Verfahren zur Identifizierung von Verbindungen, die die Expression oder Aktivität von Proteinen, die für eine neuronale Zelle letal sind, hemmen oder verbessern, wobei die Proteine normalerweise die Expression induzieren oder entweder das Gas1-Protein oder ein Protein in dem Signaltransduktionsweg, zu dem Gas1 als Bestandteil gehört, aktivieren, wobei das Verfahren:

a. die Verwendung einer neuronalen Zelle oder eines neuronalen Gewebes, die bzw. das eine für ein Gas1-Protein oder ein Gas1-Derivat, das zur Apoptoseinduktion in der Zelle fähig ist, codierende Nukleotidsequenz und ii) eine weitere, für das anderenfalls letale Protein codierende Nukleotidsequenz umfaßt,
b. die Hemmung der Funktion und/oder der Expression des Gas1-Proteins oder Gas1-Derivats oder eines Proteins in dem Apoptoseweg, zu dem Gas1 als Bestandteil gehört,
c. die Expression der für das anderenfalls letale Protein codierenden Sequenz,
d. das Inkontaktbringen der Zelle mit einer zu testenden Verbindung sowie
e. das Verfolgen des Effekts der Verbindung auf das anderenfalls letale Protein im Vergleich mit einer identischen Zelle, die nicht mit der Vebindung in Kontakt gebracht wurde, umfaßt.

**3.** Verfahren nach Anspruch 1 oder 2, wobei zur Hemmung der Expression oder Aktivität des Gas1-Proteins ein zur Hybridisierung an Gasl-DNA entsprechende mRNA zur Verhinderung der Expression davon fähiges Nukleinsäuremolekül in der Zelle bereitgestellt wird.

**4.** Verfahren nach Anspruch 1 oder 2, wobei zur Hemmung der Expression oder Aktivität des Gas1-Proteins die Expression oder Aktivität eines Proteins in dem Weg, zu dem Gas1 als Bestandteil gehört, gehemmt wird.

**5.** Verfahren nach einem der Ansprüche 1 bis 4, wobei die Zelle zur Expression des Gas1-Proteins induziert wird, indem man die Zelle mit einem für ein Gas1-Protein oder ein Gas1-Derivat, das zur Apoptoseinduktion in einer neuronalen Zelle fähig ist, codierenden induzierbaren Expressionsvektor transfiziert.

**6.** Verfahren nach Anspruch 5, wobei die Zelle zur Expression des Gas1-Proteins induziert wird, indem man die Zelle mit einem Induktionsmittel, wie beispielsweise Muristeron, in Kontakt bringt.

**7.** Verfahren nach einem der Ansprüche 1 bis 6, wobei zur Expression der weiteren, für das anderenfalls letale Protein codierenden Sequenz diese auf einem geeigneten Expressionsvektor bereitgestellt wird.

**8.** Verfahren nach einem der Ansprüche 1 bis 7, wobei es sich bei dem anderenfalls letalen Protein um ein rekombinantes Protein mit hoher Expression handelt.

**9.** Verfahren nach einem der Ansprüche 1 bis 7, wobei das anderenfalls letale Protein einen beliebigen Glutamat-, NMDA-, AMPA- oder Kainat-Rezeptor umfaßt.

**10.** Verfahren nach Anspruch 9, wobei der Glutamat-Rezeptor einen beliebigen metabotropen Rezeptor vom Typ 1 bis 8 umfaßt.

**11.** Verfahren nach einem der Ansprüche 3 bis 9, wobei das Nukleinsäuremolekül in Form eines Oligonukleotids oder eines eine Nukleotidsequenz des Nukleinsäuremoleküls enthaltenden Vektors bereitgestellt wird.

**12.** Verfahren nach Anspruch 11, wobei das Nukleinsäuremolekül ein aus der in Sequenz-ID-Nr. 5 dargestellten Nukleotidsequenz bestehendes Oligonukleotid umfaßt.

**13.** Verfahren nach Anspruch 11, wobei das Nukleinsäuremolekül ferner Ribozym- oder DNAzym-Aktivität umfaßt.

**14.** Verfahren nach einem der Ansprüche 1 bis 13, wobei das Gas1-Protein aus einem Säuger stammt.

**15.** Verfahren nach Anspruch 14, wobei das Gas1-Protein entweder aus dem Menschen, der Maus oder der Ratte stammt .

**16.** Verfahren nach Anspruch 14 oder 15, wobei das Gas1-Protein die entweder in Sequenz-ID-Nr. 2 oder 4 dargestellte Aminosäuresequenz oder ein Gas1-Derivat, das zur Apoptoseinduktion in einer neuronalen Zelle fähig ist, umfaßt.

**17.** Nukleinsäuremolekül, codierend für ein zur Apoptoseinduktion in einer neuronalen Zelle fähiges und eine Aminosäuresequenz nach Sequenz-ID-Nr. 4 umfassendes Protein, oder dazu komplementäres Nukleinsäuremolekül.

**18.** Nukleinsäuremolekül nach Anspruch 17, bei dem es sich um eine DNA-Sequenz handelt.

**19.** Nukleinsäuremolekül nach Anspruch 18, bei dem es sich um ein cDNA-Molekül handelt.

**20.** Nukleinsäuremolekül nach Anspruch 17, 18 oder 19, umfassend die Sequenz von Nukleotiden nach Sequenz-ID-Nr. 1.

**21.** Verwendung eines Antisense-Moleküls, das zur Hybridisierung an das Nukleinsäuremolekül nach einem der Ansprüche 17 bis 20 unter Bedingungen hoher Stringenz fähig ist, in einem Verfahren nach einem der Ansprüche 1 bis 16.

**22.** Verwendung eines Antisense-Moleküls nach Anspruch 21, das eine Sequenz von Nukleotiden nach Sequenz-ID-Nr. 3 oder 5 umfaßt.

**23.** Protein, das zur Apoptoseinduktion in einer neuronalen Zelle fähig ist, umfassend eine Aminosäuresequenz nach Sequenz-ID-Nr. 4.

**24.** Expressionsvektor, umfassend ein Nukleinsäuremolekül nach einem der Ansprüche 17 bis 20.

**25.** Expressionsvektor nach Anspruch 24, wobei es sich um einen von einem Plasmid, Virus oder Phagen abgeleiteten Vektor handelt.

**26.** Expressionsvektor nach Anspruch 24 oder 25, umfassend einen gewebe- oder zellspezifischen Promotor.

**27.** Expressionsvektor nach einem der Ansprüche 24 bis 26, ferner umfassend eine für ein proapoptotisches Protein codierende Sequenz.

**28.** Expressionsvektor nach einem der Ansprüche 24 bis 27, der zur Expression des Gasl-Polypeptids oder des zur Apoptoseinduktion in einer Zelle fähigen Polypeptids induziert werden kann.

**29.** Expressionsvektor nach Anspruch 28, umfassend den induzierbaren Vektor pIND.

**30.** Wirtszelle, -gewebe oder nichtmenschlicher Wirtsorganismus, transformiert, transfiziert oder infiziert mit einem Vektor nach einem der Ansprüche 24 bis 29.

**31.** In-vitro-Verfahren zur Identifizierung von Verbindungen, die zur Verhinderung oder Beschleunigung des durch Gas1 vermittelten Zelltods fähig sind, umfassend die folgenden Schritte:

a. das Inkontaktbringen einer Gas1 oder ein Gas1-Derivat, das zur Apoptoseinduktion in einer Zelle fähig ist, exprimierenden neuronalen Zelle mit der zu testenden Vebindung und
b. das Verfolgen des Effekts der Verbindung auf den Zustand der Zelle im Vergleich mit einer neuronalen Zelle, die nicht mit der Verbindung in Kontakt gebracht wurde.

**32.** Verfahren nach Anspruch 31, wobei die Zelle in Schritt (a) eine Zelle nach Anspruch 30 umfaßt.

**33.** Pharmazeutische Zusammensetzung, umfassend ein Antisense-Molekül nach Anspruch 21 oder 22 zusammen mit einem pharmazeutisch unbedenklichen Träger, Verdünnungsmittel oder Hilfsstoff dafür.

**34.** Verwendung eines Antisensemoleküls nach Anspruch 21 oder 22 bei der Herstellung eines Arzneimittels zur Vorbeugung oder Behandlung einer neurologischen Erkrankung, die wenigstens teilweise durch die Expression eines Gas1-Proteins oder eines Derivats davon, das zur Apoptoseinduktion in einer Zelle fähig ist, oder eines Proteins in dem Weg, zu dem Gas1 als Bestandteil gehört, vermittelt wird.

**35.** Verwendung nach Anspruch 34, wobei es sich bei der neurologischen Erkrankung um Morbus Parkinson, Morbus Alzheimer, Morbus Huntington, amyotrophe Lateralsklerose, ein durch Thrombose verursachtes neurologisches Leiden oder um ein Hirntrauma handelt.

**36.** Verwendung eines zur Bindung an ein Protein nach einem der Ansprüche 28 bis 30 fähigen Antikörpers in einem Verfahren nach Anspruch 2 oder 31.

## Revendications

**1.** Procédé in vitro d'inhibition de l'effet létal de l'expression d'une protéine autrement létale dans une cellule neuronale, dans lequel ladite protéine : soit induit normalement l'expression de la protéine Gas1 ou d'une protéine de la voie de transduction du signal, dont la Gas1 est un composant ; soit active celle-ci ; ledit procédé comprenant les étapes consistant à :

(a) utiliser une cellule neuronale ou un tissu neuronal ayant : (i) une séquence nucléotidique codant pour une protéine Gas1 ou un dérivé de Gas1, qui est capable d'induire l'apoptose dans ladite cellule ; et (ii) une séquence nucléotidique supplémentaire codant pour ladite protéine autrement létale ;
(b) inhiber la fonction et/ou l'expression de ladite protéine Gas1 ou d'un dérivé de Gas1 ; et
(c) exprimer ladite séquence codant pour ladite protéine autrement létale.

**2.** Procédé in vitro d'identification de composés qui inhibent ou amplifient l'expression ou l'activité de protéines qui sont létales pour une cellule neuronale, dans lequel lesdites protéines induisent normalement l'expression de la protéine Gas1 ou d'une protéine de la voie de transduction du signal, dont la Gas1 est un composant, soit activent celle-ci, ledit procédé comprenant les étapes consistant à :

(a) utiliser une cellule neuronale ou un tissu neuronal, comprenant une séquence nucléotidique codant pour une protéine Gas1 ou un dérivé de Gas1, qui est capable d'induire l'apoptose dans ladite cellule ; et (ii) une séquence nucléotidique supplémentaire codant pour ladite protéine autrement létale ;
(b) inhiber la fonction et/ou l'expression de ladite protéine Gas1, d'un dérivé de Gas1 ou d'une protéine dans la voie apoptotique dont Gas1 est un composant ;
(c) exprimer ladite séquence codant pour ladite protéine autrement létale ;
(d) mettre en contact ladite cellule avec un composé à tester ; et
(e) suivre l'effet dudit composé sur ladite protéine autrement létale en comparaison avec une cellule identique qui n'a pas été mise en contact avec ledit composé.

**3.** Procédé selon la revendication 1 ou 2, dans lequel ladite expression ou activité de la protéine Gas1 est inhibée par la fourniture d'une molécule d'acide nucléique dans ladite cellule, qui est capable de s'hybrider avec l'ARNm correspondant à l'ADN de Gas1 afin d'empêcher l'expression de celle-ci.

**4.** Procédé selon la revendication 1 ou 2, dans lequel ladite expression ou activité de ladite protéine Gas1 est inhibée par l'inhibition de l'expression ou de l'activité d'une protéine de la voie dont la Gas1 est un composant.

**5.** Procédé selon l'une quelconque des revendications 1 à 4, dans lequel ladite cellule est induite, pour exprimer ladite protéine Gas1, par une transfection de ladite cellule avec un vecteur d'expression inductible codant pour une protéine Gas1 ou un dérivé de Gas1, qui est capable d'induire l'apoptose dans une cellule neuronale.

6. Procédé selon la revendication 5, dans lequel ladite cellule est induite, pour exprimer ladite protéine Gas1, par une mise en contact de ladite cellule avec un agent d'induction, tel que la muristérone.

7. Procédé selon l'une quelconque des revendications 1 à 6, dans lequel ladite séquence supplémentaire codant pour ladite protéine autrement létale est exprimée par la fourniture de celle-ci sur un vecteur d'expression approprié.

8. Procédé selon l'une quelconque des revendications 1 à 7, dans lequel ladite protéine létale est une protéine recombinante hautement exprimée.

9. Procédé selon l'une quelconque des revendications 1 à 7, dans lequel ladite protéine autrement létale comprend un quelconque récepteur parmi ceux du glutamate, du NMDA, de l'AMPA ou du kaïnate.

10. Procédé selon la revendication 9, dans lequel lesdits récepteurs du glutamate comprennent un quelconque récepteur métabotropique des types 1 à 8.

11. Procédé selon l'une quelconque des revendications 3 à 9, dans lequel ladite molécule d'acide nucléique est fournie en tant qu'oligonucléotide ou en tant que vecteur, y compris une séquence nucléotidique de ladite molécule d'acide nucléique.

12. Procédé selon la revendication 11, dans lequel ladite molécule d'acide nucléique comprend un oligonucléotide, composé de la séquence nucléotidique représentée sur la Séquence ID n° 5.

13. Procédé selon la revendication 11, dans lequel ladite molécule d'acide nucléique comprend en outre une activité de ribozyme ou de DNAzyme.

14. Procédé selon l'une quelconque des revendications 1 à 13, dans lequel ladite protéine Gas1 est d'origine mammalienne .

15. Procédé selon la revendication 14, dans lequel ladite protéine Gas1 provient d'un quelconque être humain, souris ou rat.

16. Procédé selon la revendication 14 ou 15, dans lequel ladite protéine Gas1 comprend la séquence d'acides aminés représentée sur la Séquence ID n° 2 ou 4 ou un dérivé de Gas1, qui est capable d'induire une apoptose dans une cellule neuronale.

17. Molécule d'acide nucléique codant pour une protéine capable d'induire une apoptose dans une cellule neuronale, comprenant une séquence d'acides aminés selon la Séquence ID n° 4 ou une molécule d'acide nucléique complémentaire de celle-ci.

18. Molécule d'acide nucléique, selon la revendication 17, qui est une séquence d'ADN.

19. Molécule d'acide nucléique, selon la revendication 18, qui est une molécule d'ADNc.

20. Molécule d'acide nucléique, selon la revendication 17, 18 ou 19, comprenant la séquence de nucléotides selon la Séquence ID n° 1.

21. Utilisation d'une molécule antisens capable de s'hybrider avec une molécule d'acide nucléique, selon l'une quelconque des revendications 17 à 20, dans des conditions de stringence élevée, dans un procédé selon l'une quelconque des revendications 1 à 16.

22. Utilisation d'une molécule antisens, selon la revendication 21, comprenant une séquence de nucléotides selon la Séquence ID n° 3 ou 5.

23. Protéine capable d'induire l'apoptose dans une cellule neuronale, comprenant une séquence d'acides aminés selon la Séquence ID n° 4.

**24.** Vecteur d'expression comprenant une molécule d'acide nucléique selon l'une quelconque des revendications 17 à 20.

**25.** Vecteur d'expression selon la revendication 24, dans laquelle ledit vecteur est un quelconque vecteur dérivé d'un plasmide, d'un virus ou d'un phage.

**26.** Vecteur d'expression, selon la revendication 24 ou 25, comprenant un promoteur spécifique d'un tissu ou d'une cellule.

**27.** Vecteur d'expression, selon l'une quelconque des revendications 24 à 26, comprenant en outre une séquence codant pour une protéine pro-apoptotique.

**28.** Vecteur d'expression, selon l'une quelconque des revendications 24 à 27, qui est inductible pour l'expression dudit polypeptide Gas1 ou dudit polypeptide capable d'induire l'apoptose dans une cellule.

**29.** Vecteur d'expression, selon la revendication 28, comprenant le vecteur inductible pIND.

**30.** Cellule hôte, tissu ou organisme non humain transformée, transfectée ou infectée avec un vecteur selon l'une quelconque des revendications 24 à 29.

**31.** Procédé in vitro d'identification de composés capables d'empêcher ou d'accélérer la mort d'une cellule par médiation de Gas1, comprenant les étapes consistant à :

(a) mettre en contact une cellule neuronale, exprimant une Gas1 ou un dérivé de Gas1, capable d'induire l'apoptose dans une cellule, avec ledit composé à tester ; et
(b) suivre l'effet dudit composé sur l'état de ladite cellule en comparaison avec une cellule neuronale qui n'a pas été mise en contact avec ledit composé.

**32.** Procédé selon la revendication 31, dans lequel ladite cellule de l'étape (a) comprend une cellule selon la revendication 30.

**33.** Composition pharmaceutique comprenant une molécule antisens, selon la revendication 21 ou 22, conjointement avec un transporteur, diluant ou excipient de celle-ci acceptable d'un point de vue pharmaceutique.

**34.** Utilisation d'une molécule antisens, selon la revendication 21 ou 22, dans la fabrication d'un médicament destiné à prévenir ou à traiter un trouble neurologique par médiation, au moins en partie, de l'expression d'une protéine Gas1, d'un dérivé de celle-ci capable d'induire l'apoptose dans une cellule ou d'une protéine de la voie dont la Gas1 est un composant.

**35.** Utilisation selon la revendication 34, dans laquelle ledit trouble neurologique est n'importe lequel parmi : la maladie de Parkinson ; la maladie d'Alzheimer ; la maladie de Huntington ; une sclérose latérale amyotrophique ; une condition neurologique provoquée par une thrombose ; ou un traumatisme cérébral.

**36.** Utilisation d'un anticorps capable de se lier à une protéine, selon l'une quelconque des revendications 28 à 30, dans un procédé selon les revendications 2 ou 31.

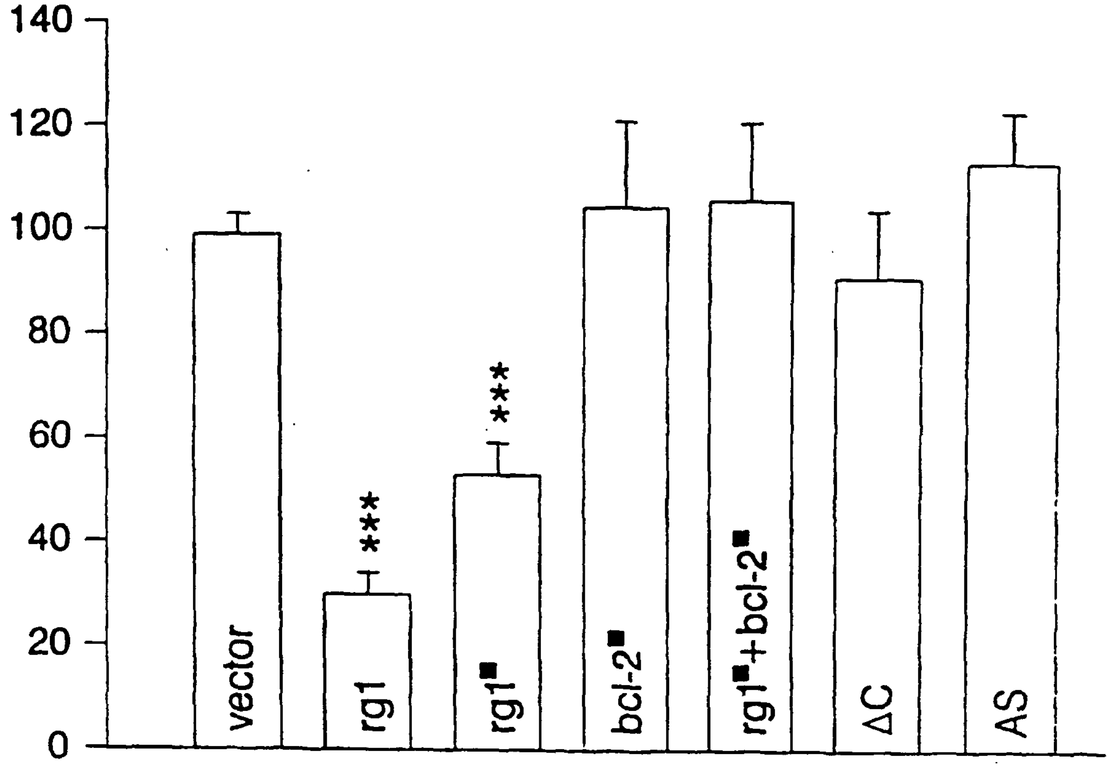


FIG. 1

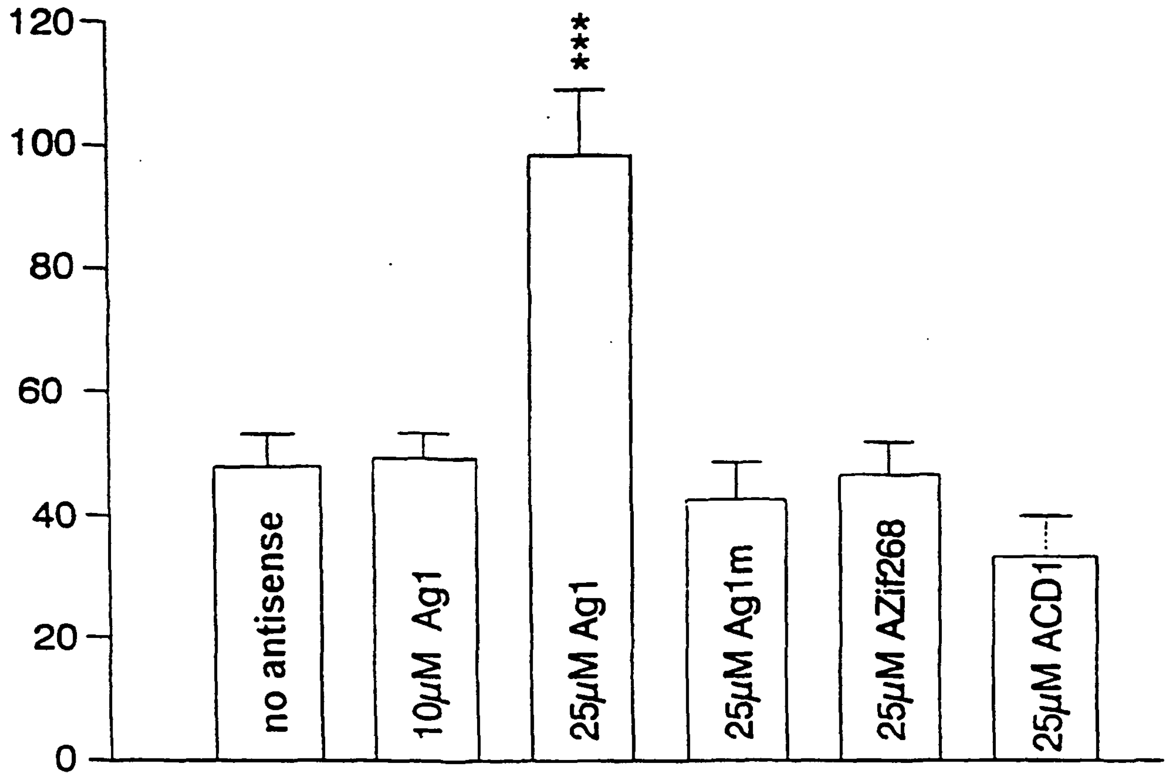
120
100
80
60
40
20
0
***
no antisense
10μM Ag1
25μM Ag1
25μM Ag1m
25μM AZif268
25μM ACD1

FIG. 2A

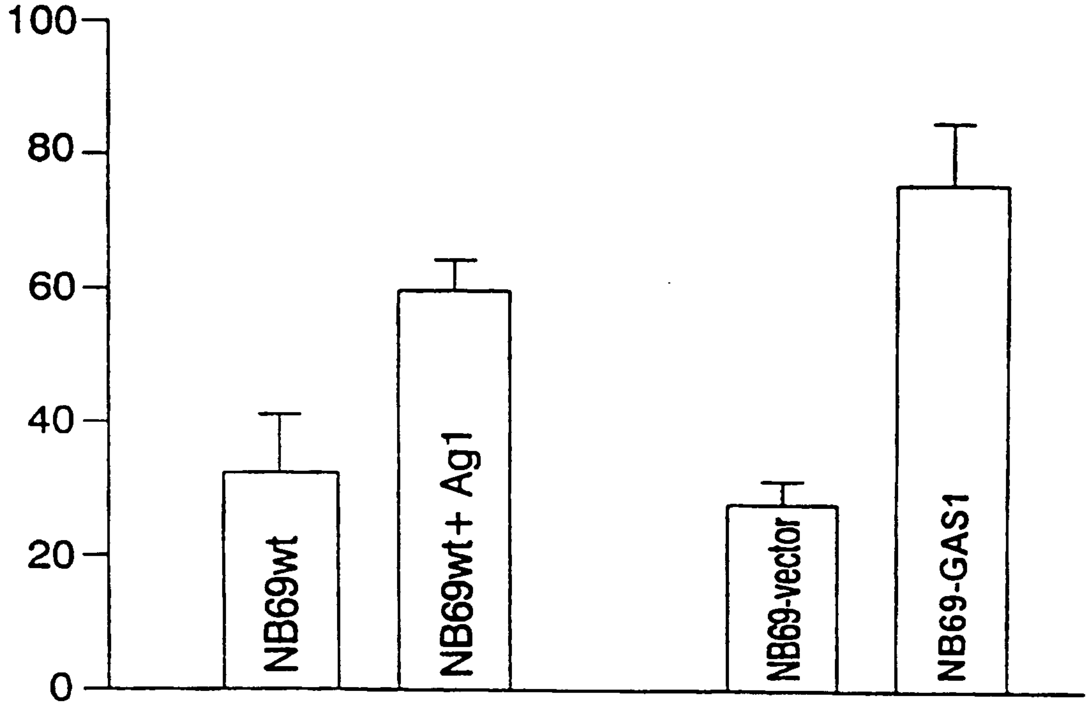


FIG. 2B

FIGURE 3

A

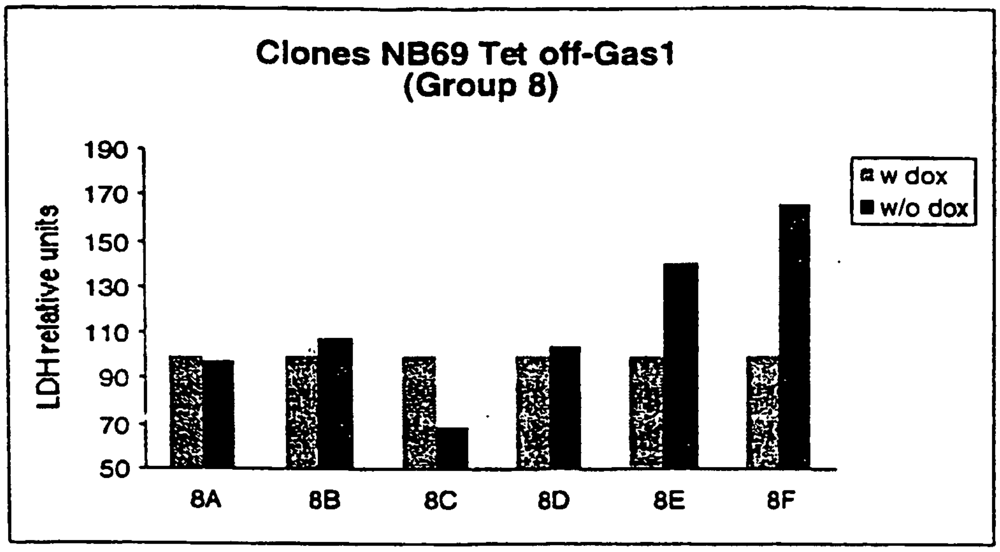
Clones NB69 Tet off-Gas1
(Group 8)
LDH relative units
190
170
150
130
110
90
70
50
w dox
w/o dox
8A
8B
8C
8D
8E
8F

B

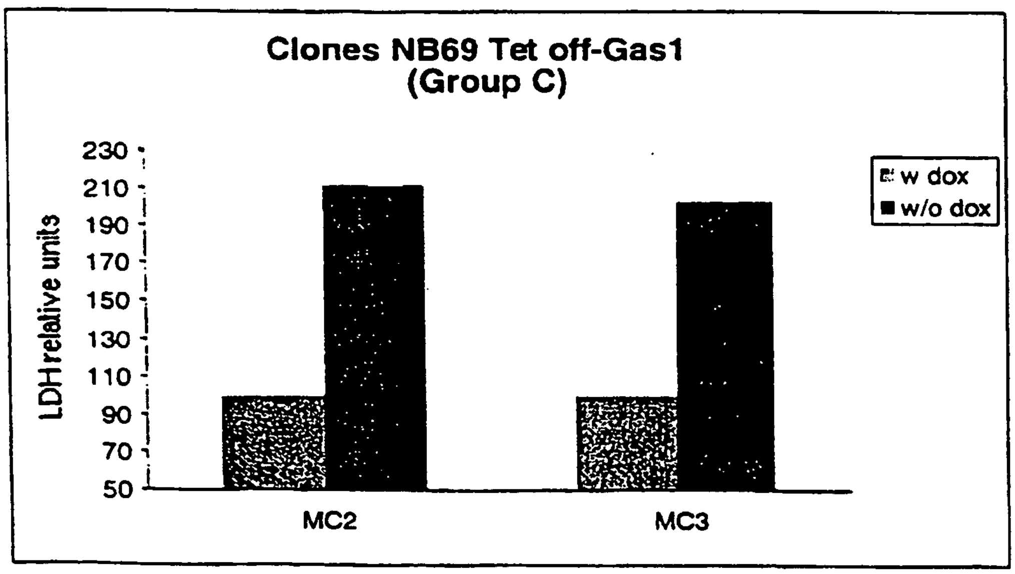
Clones NB69 Tet off-Gas1
(Group C)
LDH relative units
230
210
190
170
150
130
110
90
70
50
w dox
w/o dox
MC2
MC3

Figure 4

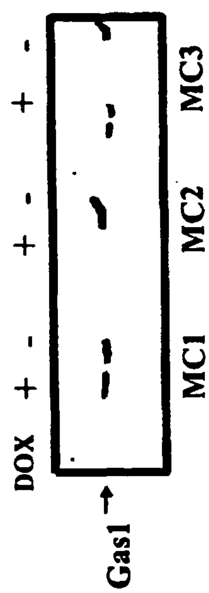

Figure 5

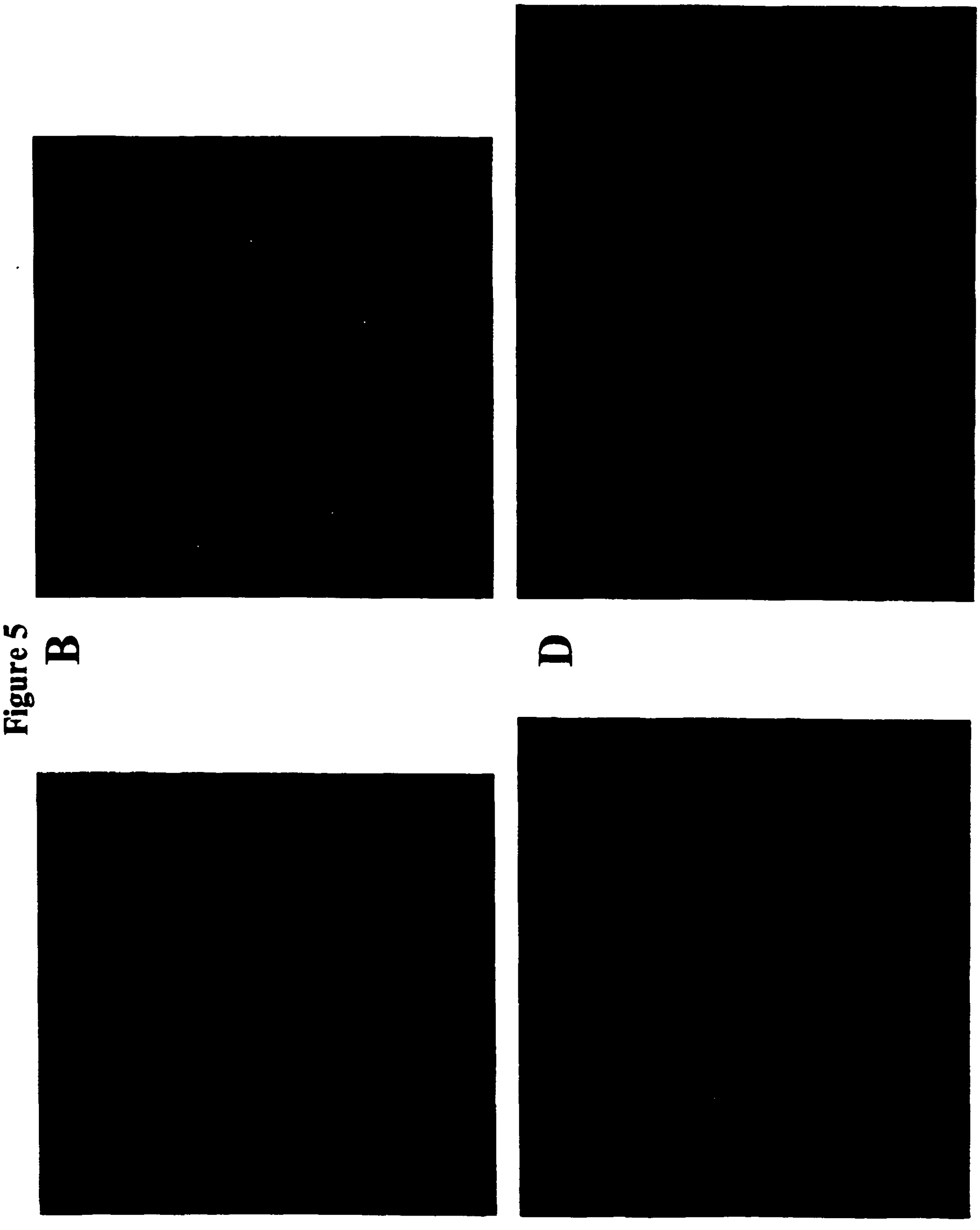

A B C D

# Figure 6

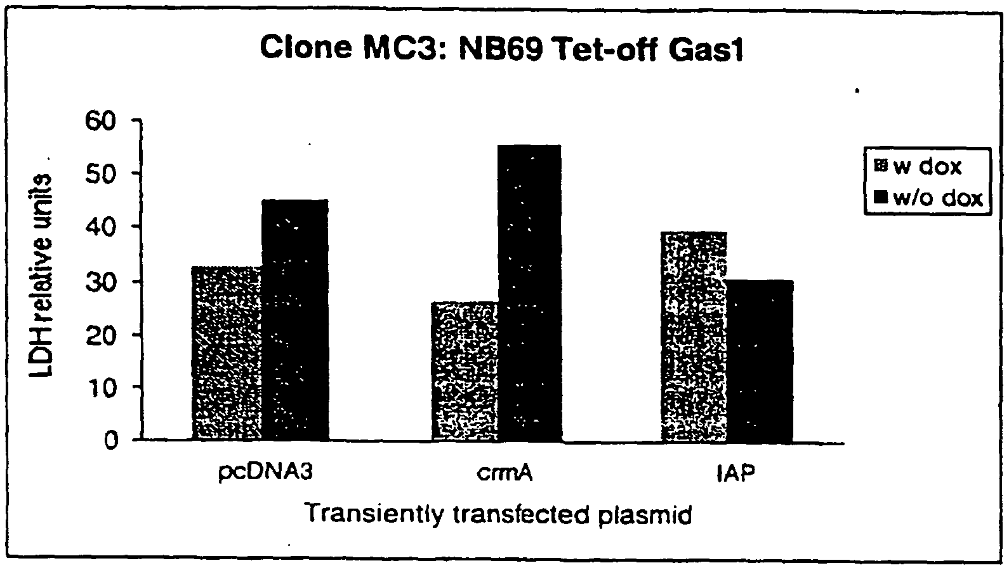
Clone MC3: NB69 Tet-off Gas1
LDH relative units
60
50
40
30
20
10
0
w dox
w/o dox
pcDNA3
crmA
IAP
Transiently transfected plasmid

# Figure 7

A

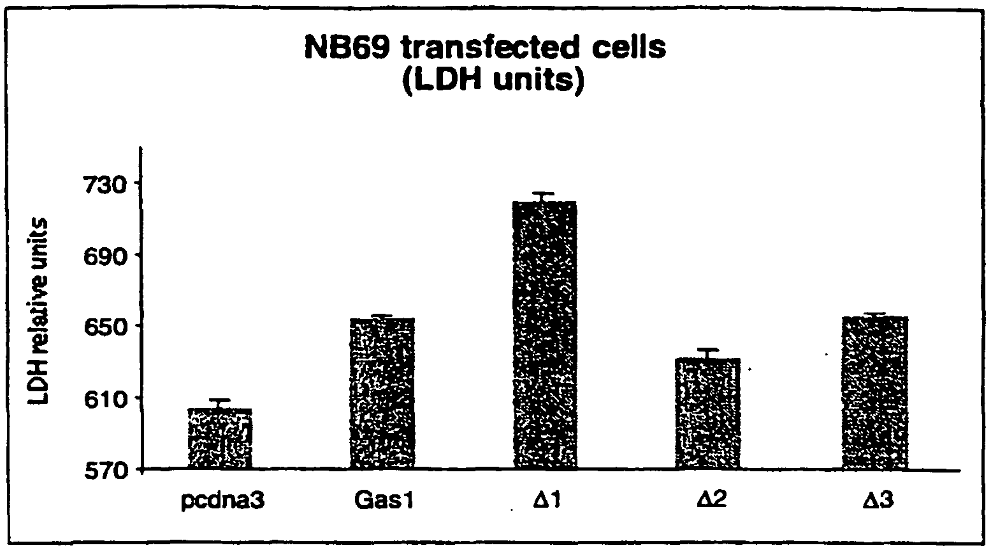
NB69 transfected cells
(LDH units)
LDH relative units
730
690
650
610
570
pcdna3
Gas1
Δ1
Δ2
Δ3

B

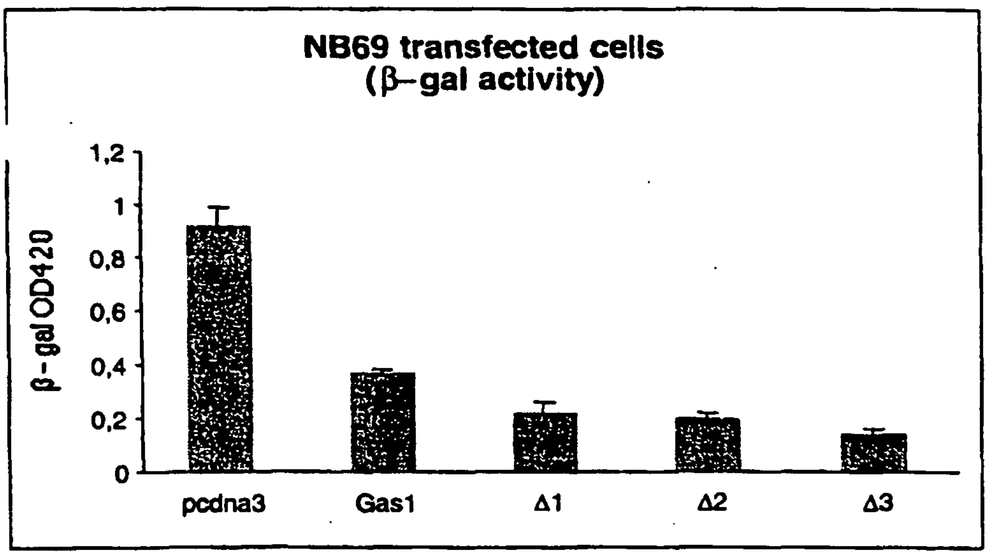
NB69 transfected cells
(β-gal activity)
β-gal OD420
1,2
1
0,8
0,6
0,4
0,2
0
pcdna3
Gas1
Δ1
Δ2
Δ3

Figure 8

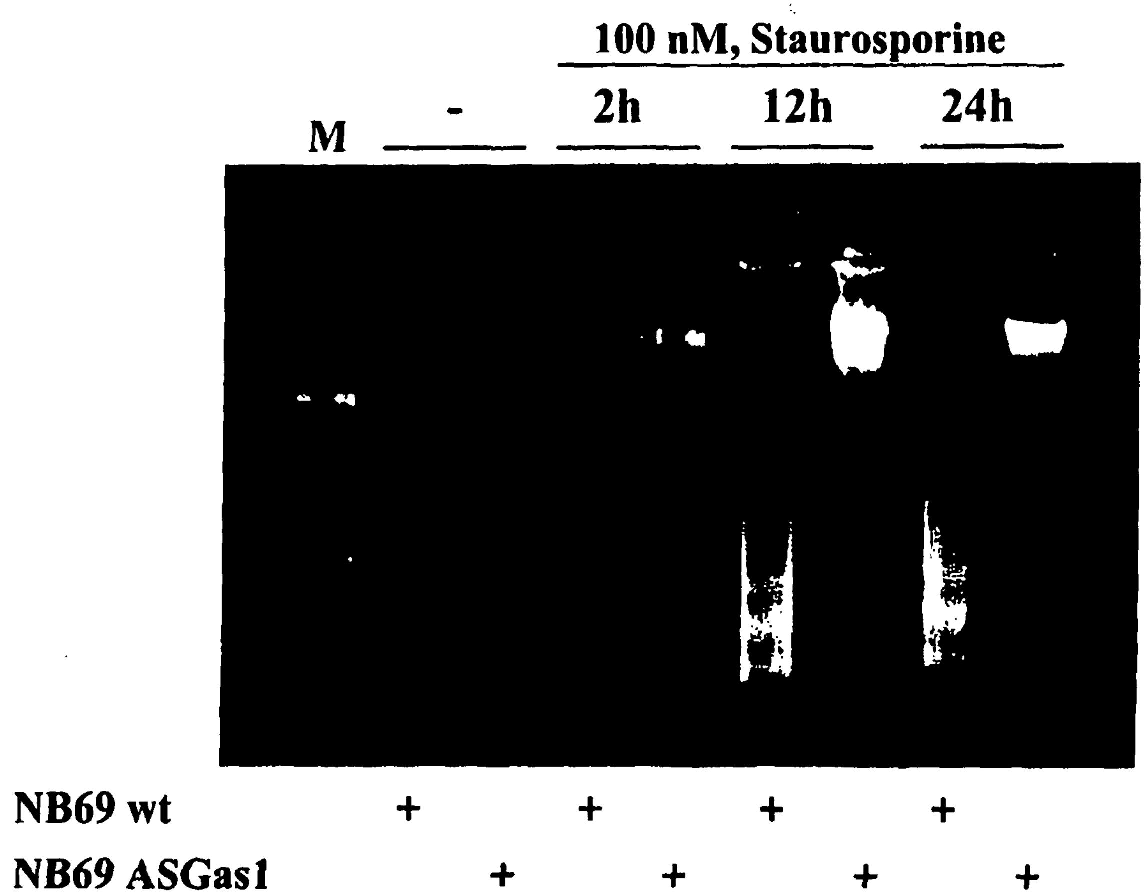

# Figure 9

A

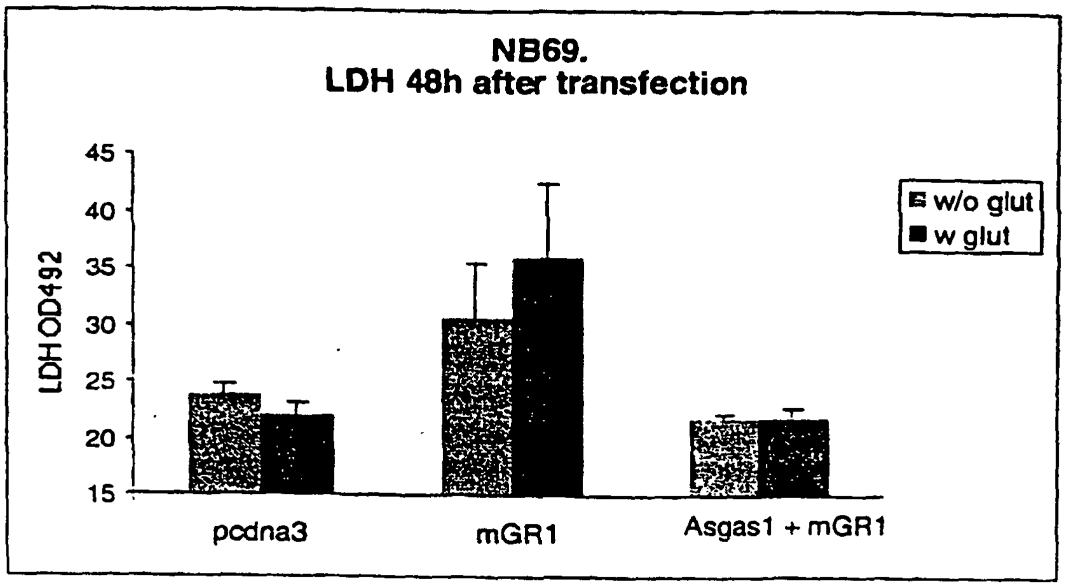
NB69.
LDH 48h after transfection
LDH OD492
45
40
35
30
25
20
15
w/o glut
w glut
pcdna3
mGR1
Asgas1 + mGR1

B

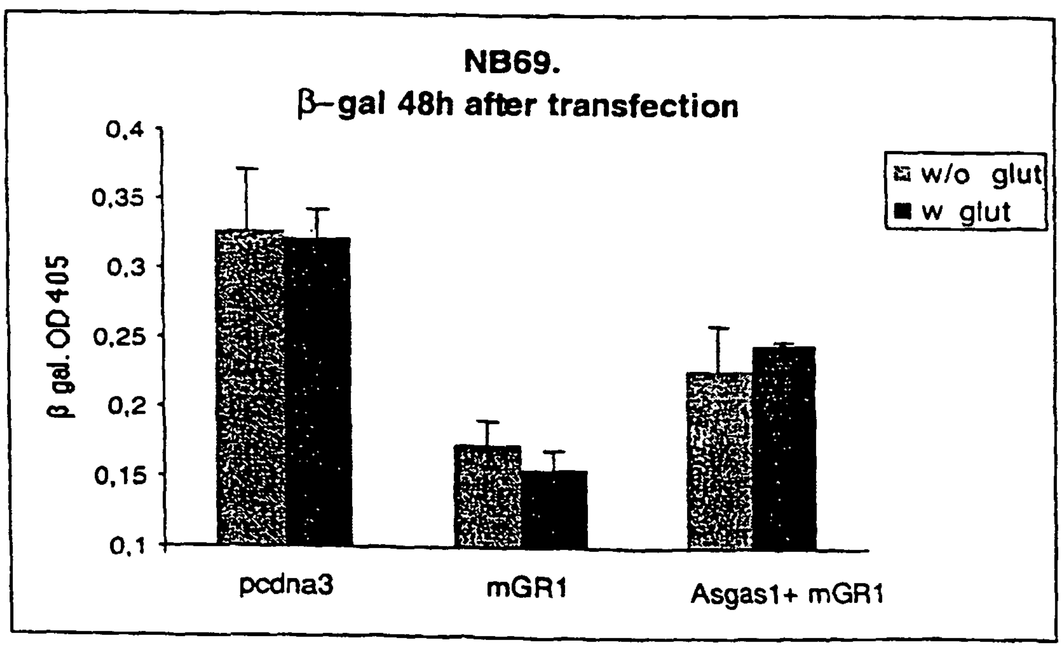
NB69.
β-gal 48h after transfection
β gal. OD405
0,4
0,35
0,3
0,25
0,2
0,15
0,1
w/o glut
w glut
pcdna3
mGR1
Asgas1+ mGR1

SEQUENCE LISTING

<110> Janssen Pharmaceutica N.V

<120> GAS1 POLYPEPTIDES

<130> 53077/000

<140>
<141>

<160> 11

<170> PatentIn Ver. 2.0

<210> 1
<211> 1762
<212> DNA
<213> Rattus norvegicus

<400> 1

```
agaaacgctc cgagtttcgg ggtcctccct gcgcctggct cccagaactt cccggagtgc 60
ggcccaggcc cgggtagagg gggaggggac caagcgtcct ggtcgcggga aggcgggatg 120
cagagctgcg aactgctacg cgctactgag ccgggaacga ggagccaccg gccacacgac 180
cttctgcagg cgccttgcac catgcacccc cggcgccgcg gctcctgcag ccttgccgcg 240
tcccccaggg acagcactga acgcttccag ccgccgatcg gagctggacc tgcgtcgccg 300
cgcctctcag ggagccccgg ccgcggtgct ggagcaaaca cccgcctcta ccagccgcgg 360
ggccgccggc atggatggat gaggacgccc atgccagaag tgcgcggaac tcggacaaac 420
ttttccagcg gccccgcggc cgccacccga gcctcgtttc cgctccgcac cgcgtccggc 480
ggccgctgct gcccgcgatg ctagccgcac tgctggcggc cggcggagcc cgctcgggga 540
gcttgccggg cgccttgctg tgcctgatgg cgctgctgca gttgctgggc tcggcgccgc 600
ggggctcggg gctggcgcac ggccgccgcc tcatctgctg gcaggcgctg ctgcagtgtc 660
agggagagcc ggactgcagc tacgcctaca gccagtacgc cgaggcgtgt gcgccggtgc 720
tcgcacagcg cggcggagct gatgctcctg ggcccgtcgg cgccttcccc tcctcggccg 780
cgtccttctc gccgcgctgg cgatgcccga gccactgcat ctcggcgctc atccagctca 840
accacacgcg ccgcgggccc gcgctggagg actgcgactg cgcgcaggac gaacactgca 900
ggtccaccaa gcgcgccatc gagccctgtc tgccgcgcac cagcagcgtg ggcccgggcg 960
cgggagcggg ctcggtcatg ggctgcaccg aggcccggcg gcgctgcgac cgggacagcc 1020
gctgcaacct ggcactcggc cgctatctag cctactgcgg caagcttttc aacggtctcc 1080
gccgcaccga cgagtgccgc gcggtcatcg aggacatgct ggcggtgccc aaggcggcgc 1140
tgctcaacga ctgtgtgtgc gatgggctgg agaggcccat ctgcgagtcg gtcaaggaga 1200
acatggcccg cctgtgcttc ggcccagatg cgagcaacgg tccgggcagc agcggctcgg 1260
acgggggcct agacgactac tacgacgaag aatatgatga cgagcagcgc gctggggccg 1320
cgggcggcga gcagcccctg gacgacgacg acggccttgc gcgcccgggc ggcggcgctg 1380
ctggggcagg cggacgcggg gacttgcccc acgggccggg gcgcaggagc agcagcagcg 1440
gcagtggagg ccactgggcg acccgaagct cctggacccc gtttgcctgt tttctgctgc 1500
tcctgctgct tgggtcccac ttgtagcccc cgcgcccctg ctgcaggcta ctccaggccg 1560
gtacgcgcga cccagctgat tcccgagcag ggcacctgtg gctcggagac cggagatgat 1620

caggagcact gacccacact ctccgcgggt agacagggcc cagcccgtac cgcaactacc 1680
atgctcggcc cttttctgtg cggttttcca ggctgcagat aaaaccggcc gatctatact 1740
gccggctcta atctgcagaa tt                                          1762
```

<210> 2
<211> 383
<212> PRT
<213> Rattus norvegicus

<400> 2

```
Met Asp Glu Asp Ala His Ala Arg Ser Ala Arg Asn Ser Asp Lys Leu
1               5                   10                  15

Phe Gln Arg Pro Arg Gly Arg His Pro Ser Leu Val Ser Ala Pro His
            20                  25                  30

Arg Val Arg Arg Pro Leu Leu Pro Ala Met Leu Ala Ala Leu Leu Gly
        35                  40                  45

Gly Gly Gly Ala Arg Ser Gly Ser Leu Pro Gly Ala Leu Leu Cys Leu
    50                  55                  60

Met Ala Leu Leu Gln Leu Leu Gly Ser Ala Pro Arg Gly Ser Gly Leu
65                  70                  75                  80

Ala His Gly Arg Arg Leu Ile Cys Trp Gln Ala Leu Leu Gln Cys Gln
                85                  90                  95

Gly Glu Pro Asp Cys Ser Tyr Ala Tyr Ser Gln Tyr Ala Glu Ala Cys
            100                 105                 110

Ala Pro Val Leu Ala Gln Arg Gly Gly Ala Asp Ala Pro Gly Pro Val
        115                 120                 125

Gly Ala Phe Pro Ser Ser Ala Ala Ser Phe Ser Pro Arg Trp Arg Cys
    130                 135                 140

Pro Ser His Cys Ile Ser Ala Leu Ile Gln Leu Asn His Thr Arg Arg
145                 150                 155                 160

Gly Pro Ala Leu Glu Asp Ser Asp Cys Ala Gln Asp Glu His Cys Arg
                165                 170                 175

Ser Thr Lys Arg Ala Ile Glu Pro Cys Leu Pro Arg Thr Ser Ser Val
            180                 185                 190

Gly Pro Gly Ala Gly Ala Gly Ser Val Met Gly Cys Thr Glu Ala Arg
        195                 200                 205
```

Arg Arg Cys Asp Arg Asp Ser Arg Cys Asn Leu Ala Leu Gly Arg Tyr
210 215 220

Leu Ala Tyr Cys Gly Lys Leu Phe Asn Gly Leu Arg Arg Thr Asp Glu
225 230 235 240

Cys Arg Ala Val Ile Glu Asp Met Leu Ala Val Pro Lys Ala Ala Leu
245 250 255

Leu Asn Asp Cys Val Cys Asp Gly Leu Glu Arg Pro Ile Cys Glu Ser
260 265 270

Val Lys Glu Asn Met Ala Arg Leu Cys Phe Gly Pro Asp Ala Ser Asn
275 280 285

Gly Pro Gly Ser Ser Gly Ser Asp Gly Gly Leu Asp Asp Tyr Tyr Asp
290 295 300

Glu Glu Tyr Asp Asp Glu Gln Arg Ala Gly Ala Ala Gly Gly Glu Gln
305 310 315 320

Pro Leu Asp Asp Asp Asp Gly Leu Ala Arg Pro Gly Gly Gly Ala Ala
325 330 335

Gly Ala Gly Gly Arg Gly Asp Leu Pro His Gly Pro Gly Arg Arg Ser
340 345 350

Ser Ser Ser Gly Ser Gly Gly His Trp Ala Thr Arg Ser Ser Trp Thr
355 360 365

Pro Phe Ala Cys Phe Leu Leu Leu Leu Leu Leu Gly Ser His Leu
370 375 380

<210> 3
<211> 1762
<212> DNA
<213> Rattus norvegicus

<400> 3

aattctgcag attagagccg gcagtataga tcggccggtt ttatctgcag cctggaaaac 60
cgcacagaaa agggccgagc atggtagttg cggtacgggc tgggccctgt ctacccgcgg 120
agagtgtggg tcagtgctcc tgatcatctc cggtctccga gccacaggtg ccctgctcgg 180
gaatcagctg ggtcgcgcgt accggcctgg agtagcctgc agcaggggcg cgggggctac 240
aagtgggacc caagcagcag gagcagcaga aaacaggcaa acggggtcca ggagcttcgg 300
gtcgcccagt ggcctccact gccgctgctg ctgctcctgc gccccggccc gtggggcaag 360
tccccgcgtc cgcctgcccc agcagcgccg ccgcccgggc gcgcaaggcc gtcgtcgtcg 420

tccaggggct gctcgccgcc cgcggcccca gcgcgctgct cgtcatcata ttcttcgtcg 480
tagtagtcgt ctaggccccc gtccgagccg ctgctgcccg gaccgttgct cgcatctggg 540
ccgaagcaca ggcgggccat gttctccttg accgactcgc agatgggcct ctccagccca 600
tcgcacacac agtcgttgag cagcgccgcc ttgggcaccg ccagcatgtc ctcgatgacc 660
gcgcggcact cgtcggtgcg gcggagaccg ttgaaaagct tgccgcagta ggctagatag 720
cggccgagtg ccaggttgca gcggctgtcc cggtcgcagc gccgccgggc ctcggtgcag 780
cccatgaccg agcccgctcc cgcgcccggg cccacgctgc tggtgcgcgg cagacagggc 840
tcgatggcgc gcttggtgga cctgcagtgt tcgtcctgcg cgcagtcgca gtcctccagc 900
gcgggcccgc ggcgcgtgtg gttgagctgg atgagcgccg agatgcagtg gctcgggcat 960
cgccagcgcg gcgagaagga cgcggccgag gaggggaagg cgccgacggg cccaggagca 1020
tcagctccgc cgcgctgtgc gagcaccggc gcacacgcct cggcgtactg gctgtaggcg 1080
tagctgcagt ccggctctcc ctgacactgc agcagcgcct gccagcagat gaggcggcgg 1140
ccgtgcgcca gccccgagcc ccgcggcgcc gagcccagca actgcagcag cgccatcagg 1200
cacagcaagg cgcccggcaa gctccccgag cgggctccgc cgccgcccag cagtgcggct 1260
agcatcgcgg gcagcagcgg ccgccggacg cggtgcggag cggaaacgag gctcgggtgg 1320
cggccgcggg gccgctggaa aagtttgtcc gagttccgcg cacttctggc atgggcgtcc 1380
tcatccatcc atgccggcgg ccccgcggct ggtagaggcg ggtgtttgct ccagcaccgc 1440
ggccggggct ccctgagagg cgcggcgacg caggtccagc tccgatcggc ggctggaagc 1500
gttcagtgct gtccctgggg gacgcggcaa ggctgcagga gccgcggcgc cgggggtgca 1560
tggtgcaagg cgcctgcaga aggtcgtgtg gccggtggct cctcgttccc ggctcagtag 1620
cgcgtagcag ttcgcagctc tgcatcccgc cttcccgcga ccaggacgct tggtcccctc 1680
cccctctacc cgggcctggg ccgcactccg ggaagttctg ggagccaggc gcagggagga 1740
ccccgaaact cggagcgttt ct 1762

<210> 4
<211> 131
<212> PRT
<213> Rattus norvegicus

<400> 4

```
His Cys Arg Ser Thr Lys Arg Ala Ile Glu Pro Cys Leu Pro Arg Thr
 1               5                  10                  15

Ser Ser Val Gly Pro Gly Ala Gly Ala Gly Ser Val Met Gly Cys Thr
            20                  25                  30

Glu Ala Arg Arg Arg Cys Asp Arg Asp Ser Arg Cys Asn Leu Ala Leu
        35                  40                  45

Gly Arg Tyr Leu Ala Tyr Cys Gly Lys Leu Phe Asn Gly Leu Arg Arg
    50                  55                  60

Thr Asp Glu Cys Arg Ala Val Ile Glu Asp Met Leu Ala Val Pro Lys
65                  70                  75                  80

Ala Ala Leu Leu Asn Asp Cys Val Cys Asp Gly Leu Glu Arg Pro Ile
                85                  90                  95

Cys Glu Ser Val Lys Glu Asn Met Ala Arg Leu Cys Phe Gly Pro Asp
            100                 105                 110

Ala Ser Asn Gly Pro Gly Ser Ser Gly Ser Asp Gly Gly Leu Asp Asp
        115                 120                 125

Tyr Tyr Asp
    130
```

<210> 5
<211> 15
<212> DNA
<213> Rattus norvegicus

<400> 5

```
tcctcatcca tccat                                                  15
```

<210> 6
<211> 26
<212> DNA
<213> Rattus norvegicus

<400> 6

```
gaattcgaga aacgctccga gtttcg                                      26
```

<210> 7
<211> 38

<212> DNA
<213> Rattus norvegicus

<400> 7

ggatccagtt tttaatacag tttatacgac gtaccagg 38

<210> 8
<211> 15
<212> DNA
<213> Rattus norvegicus

<400> 8

tcctcatcca tccat 15

<210> 9
<211> 15
<212> DNA
<213> Rattus norvegicus

<400> 9

tcctcatcca tccat 15

<210> 10
<211> 15
<212> DNA
<213> Rattus norvegicus

<400> 10

gagctggtgt tccat 15

<210> 11
<211> 15
<212> DNA
<213> Rattus norvegicus

<400> 11

gtagttgtcc atggt 15